# EUROPÄISCHE PATENTANMELDUNG

(11) **EP 1 352 641 A2**
(43) Veröffentlichungstag der Anmeldung: **15.10.2003**
(21) Anmeldenummer: 03005758.2
(22) Anmeldetag: 14.03.2003
(51) Int. Cl.: A61K 7/48

(54) **Selbstschäumende oder schaumförmige Zubereitungen, enthaltend ein oder mehrere vorgelatinisierte, quervernetzte Stärkederivate**

(30) Priorität: 11.04.2002 DE 10216503
(71) Anmelder: Beiersdorf AG, 20253 Hamburg (DE)
(72) Erfinder: Kröpke, Rainer, 22869 Schenefeld (DE); Bleckmann, Andreas, 22926 Ahrensburg (DE); Riedel, Heidi, 22529 Hamburg (DE); Conz, Celina, 25474 Bönningstedt (DE)

(57) **Zusammenfassung**

Selbstschäumende und/oder schaumförmige kosmetische oder dermatologische Zubereitungen, welche
I. ein Emulgatorsystem, welches aus
   A. mindestens einem Emulgator A, gewählt aus der Gruppe der ganz-, teil- oder nicht neutralisierten, verzweigten und/oder unverzweigten, gesättigten und/oder ungesättigten Fettsäuren mit einer Kettenlänge von 10 bis 40 Kohlenstoffatomen,
   B. mindestens einem Emulgator B, gewählt aus der Gruppe der polyethoxylierten Fettsäurester mit einer Kettenlänge von 10 bis 40 Kohlenstoffatomen und mit einem Ethoxylierungsgrad von 5 bis 100 und
   C. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen,
   D. einem oder mehreren vorgelatinisierten, quervernetzten Stärkederivaten besteht,
II. bis zu 30 Gew.-% - bezogen auf das Gesamtgewicht der Zubereitung - einer Lipidphase, welche ein oder mehrere mittelpolare Lipide mit einer Polarität von 20 mN/m bis 30 mN/m enthält,
   und
IV.1 bis 90 Vol.-%, bezogen auf das Gesamtvolumen der Zubereitung, mindestens eines Gases, gewählt aus der Gruppe Luft, Sauerstoff, Stickstoff, Helium, Argon, Lachgas (N₂O) und Kohlendioxid (CO₂)
enthalten.

## Beschreibung

Die vorliegende Erfindung betrifft selbstschäumende und/oder schaumförmige kosmetische und dermatologische Zubereitungen, insbesondere hautpflegende kosmetische und dermatologische Zubereitungen.

Schäume bzw. schaumförmige Zubereitungen gehören zu den dispersen Systemen.

Das bei weitem wichtigste und bekannteste disperse System stellen Emulsionen dar. Emulsionen sind Zwei- oder Mehrphasensysteme von zwei oder mehr ineinander nicht oder nur wenig löslichen Flüssigkeiten. Die Flüssigkeiten (rein oder als Lösungen) liegen in einer Emulsion in einer mehr oder weniger feinen Verteilung vor, die im allgemeinen nur begrenzt stabil ist.

Schäume sind Gebilde aus gasgefüllten, kugel- oder polyederförmigen Zellen, welche durch flüssige, halbflüssige, hochviskose oder feste Zellstege begrenzt werden. Die Zellstege, verbunden über sogenannte Knotenpunkte, bilden ein zusammenhängendes Gerüst. Zwischen den Zellstegen spannen sich die Schaumlamellen (geschlossenzelliger Schaum). Werden die Schaumlamellen zerstört oder fließen sie am Ende der Schaumbildung in die Zellstege zurück, erhält man einen offenzelligen Schaum. Auch Schäume sind thermodynamisch instabil, da durch Verkleinerung der Oberfläche Oberflächenenergie gewonnen werden kann. Die Stabilität und damit die Existenz eines Schaums ist somit davon abhängig, wieweit es gelingt, seine Selbstzerstörung zu verhindern.

Kosmetische Schäume sind in der Regel dispergierte Systeme aus Flüssigkeiten und Gasen, wobei die Flüssigkeit das Dispergiermittel und das Gas die dispergierte Substanz darstellen. Schäume aus niedrigviskosen Flüssigkeiten werden temporär durch oberflächenaktive Substanzen (Tenside, Schaumstabilisatoren) stabilisiert. Solche Tensidschäume haben aufgrund ihrer großen inneren Oberfläche ein starkes Adsorptionsvermögen, welches beispielsweise bei Reinigungs- und Waschvorgängen ausgenutzt wird. Dementsprechend finden kosmetische Schäume insbesondere in den Bereichen der Reinigung, beispielsweise als Rasierschaum, und der Haarpflege Verwendung.

Zur Erzeugung von Schaum wird Gas in geeignete Flüssigkeiten eingeblasen, oder man erreicht die Schaumbildung durch heftiges Schlagen, Schütteln, Verspritzen oder Rühren der Flüssigkeit in der betreffenden Gasatmosphäre, vorausgesetzt, daß die Flüssigkeiten geeignete Tenside oder andere grenzflächenaktive Stoffe (sogenannte Schaumbildner) enthalten, die außer Grenzflächenaktivität auch ein gewisses Filmbildungsvermögen besitzen.

Kosmetische Schäume haben gegenüber anderen kosmetischen Zubereitungen den Vorteil, daß sie eine feine Verteilung von Wirkstoffen auf der Haut erlauben. Allerdings sind kosmetische Schäume in der Regel nur durch Verwendung besonderer Tenside, welche darüberhinaus oft wenig hautverträglich sind, zu erreichen.

Ein weiterer Nachteil des Standes der Technik ist es, daß derartige Schäume nur wenig stabil sind, weshalb sie üblicherweise innerhalb von etwa 24 Stunden zusammenfallen. Eine Anforderung an kosmetische Zubereitungen ist aber, daß diese eine möglichst jahrelange Stabilität besitzen. Diesem Problem wird im allgemeinen dadurch Rechnung getragen, daß der Verbraucher den eigentlichen Schaum erst bei der Anwendung mit Hilfe eines geeigneten Sprühsystems selbst erzeugt, wozu beispielsweise Sprühdosen verwendet werden können, in denen ein verflüssigtes Druckgas als Treibgas dient. Beim Öffnen des Druckventils entweicht das Treibmittel-Flüssigkeitsgemisch durch eine feine Düse, das Treibmittel verdampft und hinterläßt einen Schaum.

Auch nachschäumende kosmetische Zubereitungen sind an sich bekannt. Sie werden zunächst in fließförmiger Form aus einem Aerosolbehälter auf die Haut aufgetragen und entwickeln nach kurzer Verzögerung erst dort unter dem Einfluß des enthaltenen Nachschäummittels den eigentlichen Schaum, beispielsweise einen Rasierschaum. Nachschäumende Zubereitungen liegen oft in speziellen Ausführungsformen wie etwa nachschäumenden Rasiergelen oder dergleichen vor.

Allerdings kennt der Stand der Technik keinerlei kosmetische oder dermatologische Zubereitungen, welche bereits bei der Herstellung aufgeschäumt werden könnten und dennoch eine genügend hohe Stabilität aufweisen, um in üblicher Weise verpackt, gelagert und in den Handel gebracht zu werden.

Eine Aufgabe der vorliegenden Erfindung war also, den Stand der Technik zu bereichern und kosmetische oder dermatologische selbstschäumende und/oder schaumförmige Zubereitungen zur Verfügung zu stellen, die die Nachteile des Standes der Technik nicht aufweisen.

Die Deutsche Offenlegungsschrift DE 197 54 659 offenbart, daß Kohlendioxid ein geeigneter Wirkstoff zur Stabilisierung oder Erhöhung der epidermalen Ceramidsyntheserate ist, welcher der Stärkung der Permeabilitätsbarriere, der Verminderung des transepidermalen Wasserverlusts und der Steigerung der relativen Hautfeuchtigkeit dienen kann. Zur Behandlung der Haut wird das CO₂ beispielsweise in Wasser gelöst, mit welchem anschließend die Haut gespült wird. Allerdings kennt der Stand der Technik bislang keinerlei kosmetische oder dermatologische Grundlagen, in die ein gasförmiger Wirkstoff in ausreichender, d. h. wirksamer Konzentration eingearbeitet werden könnte.

Nach dem bisherigen Stand der Technik sind schaumförmige kosmetische Emulsionen, die sich durch einen hohen Lufteintrag auszeichnen, ohne Treibgas nicht zu formulieren bzw. technisch herzustellen. Dieses gilt insbesondere für Systeme, die auf klassischen Emulgatoren und Gelbildnern basieren und durch Scherung (Rühren, Homogenisierung) einen Schaum mit einer außerordentlich hohen Stabilität entwickeln.

Eine weitere Aufgabe der vorliegenden Erfindung war es also, kosmetische oder dermatologische Grundlagen zu finden, in die sich wirksame Mengen an gasförmigen Wirkstoffen einarbeiten lassen.

Es war überraschend und für den Fachmann nicht vorauszusehen, daß selbstschäumende und/oder schaumförmige kosmetische oder dermatologische Zubereitungen, welche
I. ein Emulgatorsystem, welches aus
   A. mindestens einem Emulgator A, gewählt aus der Gruppe der ganz-, teil- oder nicht neutralisierten, verzweigten und/oder unverzweigten, gesättigten und/oder ungesättigten Fettsäuren mit einer Kettenlänge von 10 bis 40 Kohlenstoffatomen,
   B. mindestens einem Emulgator B, gewählt aus der Gruppe der polyethoxylierten Fettsäurester mit einer Kettenlänge von 10 bis 40 Kohlenstoffatomen und mit einem Ethoxylierungsgrad von 5 bis 100 und
   C. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen,
   besteht,
II. bis zu 30 Gew.-% - bezogen auf das Gesamtgewicht der Zubereitung - einer Lipidphase,
III. ein oder mehrere vorgelatinisierte, quervernetzte Stärkederivate und
IV.1 bis 90 Vol.-%, bezogen auf das Gesamtvolumen der Zubereitung, mindestens eines Gases, gewählt aus der Gruppe Luft, Sauerstoff, Stickstoff, Helium, Argon, Lachgas (N₂O) und Kohlendioxid (CO₂)
   enthalten,
den Nachteilen des Standes der Technik abhelfen.

Unter "selbstschäumend" bzw. "schaumförmig" ist im Sinne der vorliegenden Erfindung zu verstehen, daß die Gasbläschen (beliebig) verteilt in einer (oder mehreren) flüssigen Phase(n) vorliegen, wobei die Zubereitungen makroskopisch nicht notwendigerweise das Aussehen eines Schaumes haben müssen. Erfindungsgemäße selbstschäumende und/oder schaumförmige kosmetische oder dermatologische Zubereitungen können z. B. makroskopisch sichtbar dispergierte Systeme aus in Flüssigkeiten dispergierten Gasen darstellen. Der Schaumcharakter kann aber beispielsweise auch erst unter einem (Licht-) Mikroskop sichtbar werden. Darüber hinaus sind erfindungsgemäße selbstschäumende und/oder schaumförmige Zubereitungen - insbesondere dann, wenn die Gasbläschen zu klein sind, um unter einem Lichtmikroskop erkannt zu werden - auch an der starken Volumenzunahme des Systems erkennbar.

Die erfindungsgemäßen Zubereitungen stellen in jeglicher Hinsicht überaus befriedigende Präparate dar. Es war insbesondere überraschend, daß die erfindungsgemäßen schaumförmigen Zubereitungen - auch bei einem ungewöhnlich hohen Gasvolumen - außerordentlich stabil sind. Dementsprechend eignen sie sich ganz besonders, um als Grundlage für Zubereitungsformen mit vielfältigen Anwendungszwecken zu dienen. Die erfindungsgemäßen Zubereitungen zeigen sehr gute sensorische Eigenschaften, wie beispielsweise die Verteilbarkeit auf der Haut oder das Einzugsvermögen in die Haut, und zeichnen sich darüberhinaus durch eine überdurchschnittlich gute Hautpflege aus.

Durch vorgelatinisierte, quervernetzte Stärkederivate wird der Eintrag der Gase unterstützt sowie über eine längere Lagerdauer auch bei höheren Temperaturen (z.B. 40°C) ein stabilisierender sowie deutlich nachschäumender Effekt erzielt, auch ohne nach dem Stand der Technik übliche Nachschäummittel wie z.B. Treibgase zu enthalten.

Hierdurch ist es möglich, Zubereitungen mit einer herausragenden, neuartigen kosmetischen Leistung und mit außerordentlich hohem Gasvolumen über lange Lagerdauer bei hohen Temperaturen stabil zu halten. Gleichzeitig zeichnen sie sich erfindungsgemäße Zubereitungen durch eine überdurchschnittliche gute Hautpflege sowie sehr guten sensorischen Eigenschaften aus.

Gegenstand der Erfindung ist ferner
die Verwendung selbstschäumender und/oder schaumförmiger kosmetischer oder dermatologischer Zubereitungen, welche
I. ein Emulgatorsystem, welches aus
   A. mindestens einem Emulgator A, gewählt aus der Gruppe der ganz-, teil- oder nicht neutralisierten, verzweigten und/oder unverzweigten, gesättigten und/oder ungesättigten Fettsäuren mit einer Kettenlänge von 10 bis 40 Kohlenstoffatomen,
   B. mindestens einem Emulgator B, gewählt aus der Gruppe der polyethoxylierten Fettsäurester mit einer Kettenlänge von 10 bis 40 Kohlenstoffatomen und mit einem Ethoxylierungsgrad von 5 bis 100 und
   C. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen besteht,
   D. einem oder mehreren vorgelatinisierten, quervernetzten Stärkederivaten
   und
II. bis zu 30 Gew.-% - bezogen auf das Gesamtgewicht der Zubereitung - einer Lipidphase, welche ein oder mehrere mittelpolare Lipide mit einer Polarität von 20 mN/m bis 30 mN/m enthält,
enthalten, als kosmetische oder dermatologische Grundlagen für gasförmige Wirkstoffe.

Der oder die Emulgatoren A werden vorzugsweise gewählt aus der Gruppe der Fettsäuren, welche ganz oder teilweise mit üblichen Alkalien (wie z. B. Natrium- und/oder Kaliumhydroxid, Natrium- und/oder Kaliumcarbonat sowie Mono- und/oder Triethanolamin) neutralisiert sind. Besonders vorteilhaft sind beispielsweise Stearinsäure und Stearate, Isostearinsäure und Isostearate, Palmitinsäure und Palmitate sowie Myristinsäure und Myristate.

Der oder die Emulgatoren B werden vorzugsweise gewählt aus der folgenden Gruppe: PEG-9-Stearat, PEG-8-Distearat, PEG-20-Stearat, PEG-8 Stearat, PEG-8-Oleat, PEG-25-Glyceryltrioleat, PEG-40-Sorbitanlanolat, PEG-15-Glycerylricinoleat, PEG-20-Glycerylstearat, PEG-20-Glycerylisostearat, PEG-20-Glyceryloleat, PEG-20-Stearat, PEG-20-Methylglucosesesquistearat, PEG-30-Glycerylisostearat, PEG-20-Glyceryllaurat, PEG-30-Stearat, PEG-30-Glycerylstearat, PEG-40-Stearat, PEG-30-Glyceryllaurat, PEG-50-Stearat, PEG-100-Stearat, PEG-150-Laurat. Besonders vorteilhaft sind beispielsweise polyethoxylierte Stearinsäureester.

Der oder die Coemulgatoren C werden erfindungsgemäß vorzugsweise aus der folgenden Gruppe gewählt: Butyloctanol, Butyldecanol, Hexyloctanol, Hexyldecanol, Octyldodecanol, Behenylalkohol (C₂₂H₄₅OH), Cetearylalkohol [eine Mischung aus Cetylalkohol (C₁₆H₃₃OH) und Stearylalkohol (C₁₈H₃₇OH)], Lanolinalkohole (Wollwachsalkohole, die die unverseifbare Alkoholfraktion des Wollwachses darstellen, die nach der Verseifung von Wollwachs erhalten wird). Besonders bevorzugt sind Cetyl- und Cetylstearylalkohol.

Es ist erfindungsgemäß vorteilhaft, die Gewichtsverhältnisse von Emulgator A zu Emulgator B zu Coemulgator C (A : B : C) wie a : b : c zu wählen, wobei a, b und c unabhängig voneinander rationale Zahlen von 1 bis 5, bevorzugt von 1 bis 3 darstellen können. Insbesondere bevorzugt ist ein Gewichtsverhältnis von etwa 1 : 1 : 1.

Es ist vorteilhaft im Sinne der vorliegenden Erfindung, die Gesamtmenge der Emulgatoren A und B und des Coemulgators C aus dem Bereich von 2 bis 20 Gew.-%, vorteilhaft von 5 bis 15 Gew.-%, insbesondere von 8 bis 13 Gew.-%, jeweils bezogen auf das Gesamtgewicht der Formulierung, zu wählen.

Besonders bevorzugt im Sinne der vorliegenden Erfindung ist es, wenn die Gasphase der Zubereitungen Kohlendioxid enthält bzw. ganz aus Kohlendioxid besteht. Es ist insbesondere vorteilhaft, wenn Kohlendioxid einen oder den Wirkstoff in den erfindungsgemäßen Zubereitungen darstellt.

Erfindungsgemäße Zusammensetzungen entwickeln sich bereits während ihrer Herstellung - beispielsweise während des Rührens oder bei der Homogenisierung - zu feinblasigen Schäumen. Erfindungsgemäß sind feinblasige, reichhaltige Schäume von hervorragender kosmetischer Eleganz erhältlich. Weiterhin sind erfindungsgemäß besonders gut hautverträgliche Zubereitungen erhältlich, wobei wertvolle Inhaltsstoffe besonders gut auf der Haut verteilt werden können.

Es ist gegebenenfalls vorteilhaft, wenngleich nicht notwendig, wenn die Formulierungen gemäß der vorliegenden Erfindung weitere Emulgatoren enthalten. Vorzugsweise sind solche Emulgatoren zu verwenden, welche zur Herstellung von W/O-Emulsionen geeignet sind, wobei diese sowohl einzeln als auch in beliebigen Kombinationen miteinander vorliegen können.

Vorteilhaft werden der oder die weiteren Emulgatoren aus der Gruppe gewählt, die die folgenden Verbindungen umfaßt:
Polyglyceryl-2-Dipolyhydroxystearat, PEG-30-Dipolyhydroxystearat, Cetyldimethiconcopolyol, Glykoldistearat, Glykoldilaurat, Diethylenglykoldilaurat, Sorbitantrioleat, Glykololeat, Glyceryldilaurat, Sorbitantristearat, Propylenglykolstearat, Propylenglykollaurat, Propylenglykoldistearat, Sucrosedistearat, PEG-3 Castor Oil, Pentaerythritylmonostearat, Pentaerythritylsesquioleat, Glyceryloleat, Glycerylstearat, Glyceryldiisostearat, Pentaerythritylmonooleat, Sorbitansesquioleat, Isostearyldiglycerylsuccinat, Glycerylcaprat, Palm Glycerides, Cholesterol, Lanolin, Glyceryloleat (mit 40 % Monoester), Polyglyceryl-2-Sesquiisostearat, Polyglyceryl-2-Sesquioleat, PEG-20 Sorbitan Beeswax, Sorbitanoleat, Sorbitanisostearat, Trioleylphosphat, Glyceryl Stearate und Ceteareth-20 (Teginacid von Th. Goldschmidt), Sorbitanstearat, PEG-7 Hydrogenated Castor Oil, PEG-5-Soyasterol, PEG-6 Sorbitan Beeswax, Glycerylstearat SE, Methylglucosesesquistearate, PEG-10 Hydrogenated Castor Oil, Sorbitanpalmitat, PEG-22/Dodecylglykol Copolymer, Polyglyceryl-2-PEG-4-Stearat, Sorbitanlaurat, PEG-4-Laurat, Polysorbat 61, Polysorbat 81, Polysorbat 65, Polysorbat 80, Triceteareth-4-Phosphat, Triceteareth-4 Phosphate und Sodium C₁₄₋₁₇ Alkyl Sec Sulfonat (Hostacerin CG von Hoechst), Glycerylstearat und PEG-100 Stearate (Arlacel 165 von ICI), Polysorbat 85, Trilaureth-4-Phosphat, PEG-35 Castor Oil, Sucrosestearat, Trioleth-8-Phosphat, C₁₂₋₁₅ Pareth-12, PEG-40 Hydrogenated Castor Oil, PEG-16 Soya Sterol, Polysorbat 80, Polysorbat 20, Polyglyceryl-3-methylglucose Distearat, PEG-40 Castor Oil, Natriumcetearylsulfat, Lecithin, Laureth-4-Phosphat, Propylenglykolstearat SE, PEG-25 Hydrogenated Castor Oil, PEG-54 Hydrogenated Castor Oil, Glycerylstearat SE, PEG-6 Caprylic/Capric Glycerides, Glyceryloleat und Propylenglykol, Glyceryllanolat, Polysorbat 60, Glycerylmyristat, Glycerylisostearat und Polyglyceryl-3 Oleat, Glyceryllaurat, PEG-40-Sorbitanperoleat, Laureth-4, Glycerinmonostearat, Isostearylglycerylether, Cetearyl Alcohol und Natriumcetearylsulfat, PEG-22-Dodecylglykolcopolymer, Polyglyceryl-2-PEG-4-Stearat, Pentaerythrithylisostearat, Polyglyceryl-3-Diisostearat, Sorbitanoleat und Hydrogenated Castor Oil und Cera alba und Stearinsäure, Natriumdihydroxycetylphosphat und Isopropylhydroxycetylether, Methylglucosesesquistearat, Methylglucosedioleat, Sorbitanoleat und PEG-2 Hydrogenated Castor Oil und Ozokerit und Hydrogenated Castor Oil, PEG-2 Hydrogenated Castor Oil, PEG-45-/Dodecylglykolcopolymer, Methoxy PEG-22-/Dodecylglykolcopolymer, Hydrogenated Coco Glycerides, Polyglyceryl-4-lsostearat, PEG-40-Sorbitanperoleat, PEG-40-Sorbitanperisostearat, PEG-8-Beeswax, Laurylmethiconcopolyol, Polyglyceryl-2-Laurat, Stearamidopropyl-PG-dimoniumchloridphosphat, PEG-7 Hydrogenated Castor Oil, Triethylcitrat, Glycerylstearatcitrat, Cetylphosphat, Polyglycerolmethylglucosedistearat, Poloxamer 101, Kaliumcetylphosphat, Glycerylisostearat, Polyglyceryl-3-Diisostearate.

Bevorzugt werden der oder die weiteren Emulgatoren im Sinne der vorliegenden Erfindung aus der Gruppe der hydrophilen Emulgatoren gewählt. Erfindungsgemäß besonders bevorzugt sind Mono-, Di-, Trifettsäureestern des Sorbitols.

Die Gesamtmenge der weiteren Emulgatoren wird erfindungsgemäß vorteilhaft kleiner als 5 Gew.-%, bezogen auf das Gesamtgewicht der Formulierung, gewählt.

Die Liste der genannten weiteren Emulgatoren, die im Sinne der vorliegenden Erfindung eingesetzt werden können, soll selbstverständlich nicht limitierend sein.

Besonders vorteilhafte selbstschäumende und/oder schaumförmige Zubereitungen im Sinne der vorliegenden Erfindung sind frei von Mono- oder Diglycerylfettsäureestern. Insbesondere bevorzugt sind erfindungsgemäße Zubereitungen, welche kein Glycerylstearat, Glycerylisostearat, Glyceryldiisostearat, Glyceryloleat, Glycerylpalmitat, Glycerylmyristat, Glyceryllanolat und/oder Glyceryllaurat enthalten.

Besonders vorteilhafte mittelpolare Lipide im Sinne der vorliegenden Erfindung sind die im folgenden aufgelisteten.

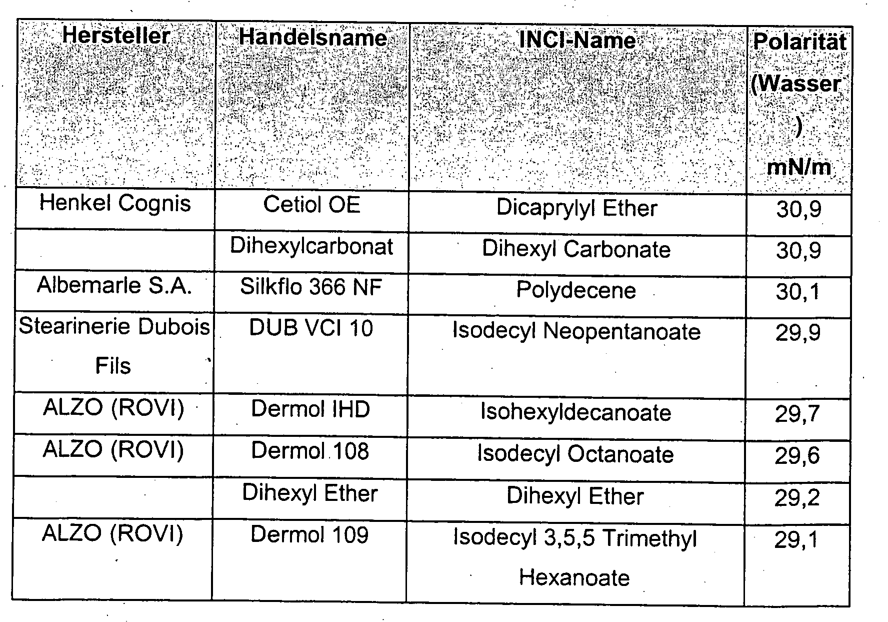

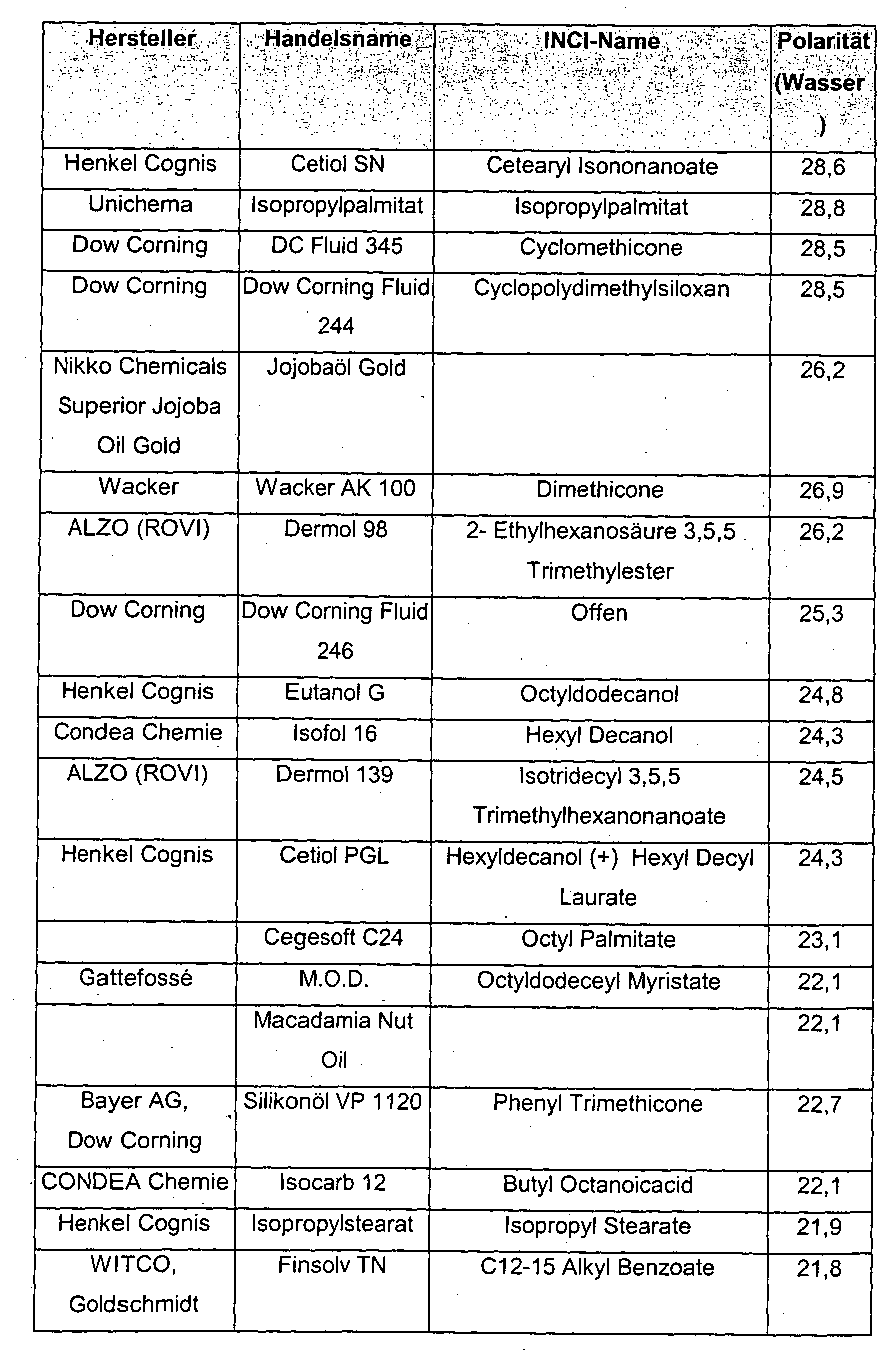

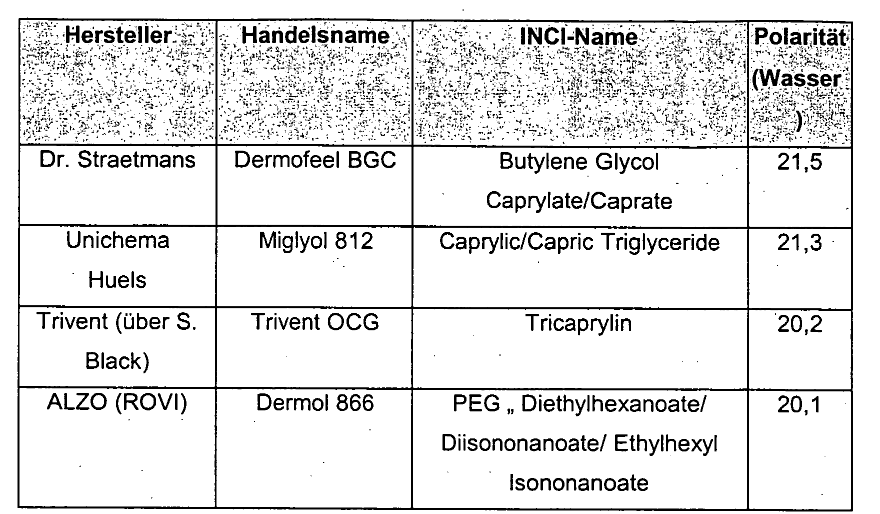

Die Gehalt der Lipidphase wird vorteilhaft kleiner als 30 Gew.-% gewählt, bevorzugt zwischen 2,5 und 30 Gew.-%, insbesondere bevorzugt zwischen 5 und 15 Gew.-%, jeweils bezogen auf das Gesamtgewicht der Zubereitung. Es ist gegebenenfalls ferner vorteilhaft, wenngleich nicht zwingend, wenn die Lipidphase bis zu 40 Gew.-% - bezogen auf das Gesamtgewicht der Lipidphase - an unpolaren Lipiden (mit einer Polarität ≥ 30 mN/m) und/oder polaren Lipiden (mit einer Polarität ≤ 20 mN/m) enthält.

Von den Kohlenwasserstoffen sind insbesondere Paraffinöl sowie weitere hydrierte Polyolefine wie hydriertes Polyisobutene, Squalan und Squalen vorteilhaft im Sinne der vorliegenden Erfindung zu verwenden.

Es ist erfindungsgemäß vorteilhaft, wenn ein oder mehrere vorgelatinisierte, quervernetzte Stärkederivate in einer Konzentration von 0,1 bis 20 Gewichts-%, bevorzugt in einer Konzentration von 0,3 bis 15 Gewichts-% und ganz besonders bevorzugt in einer Konzentration von 0,5 bis 10 Gewichts-%, bezogen auf das Gesamtgewicht der Zubereitung, in der erfindungsgemäßen Reinigungsemulsion enthalten sind.

Es ist erfindungsgemäß besonders vorteilhaft, wenn als vorgelatinisierte, quervernetzte Stärkederivate hydroxypropylierte Phosphatester eingesetzt werden. Insbesondere vorteilhaft sind solche Stärkederivate, wie sie in der US 6,248,338 beschrieben werden, besonders vorteilhaft Hydroxypropyldistärkephosphat. Ganz besonders bevorzugt ist dabei der Einsatz eines Hydroxypropyldistärkephosphates, wie es als Produkt Structure® XL der Firma National Starch verkauft wird.

Erfindungsgemäß vorteilhaft ist der Einsatz von vorgelatinisierten, quervernetzten Stärkederivaten, insbesondere von hydroxypropylierten Phosphatestern in Kombination mit anorganischen Verdickungsmitteln und / oder partikulären hydrophoben und/oder hydrophobisierten und/oder ölabsorbierenden Festkörpersubstanzen

Das oder die anorganischen Verdickungsmittel können beispielsweise vorteilhaft gewählt werden aus der Gruppe der modifizierten oder unmodifizierten, natürlich vorkommender oder synthetischer Schichtsilikate.

Es ist zwar durchaus günstig, reine Komponenten einzusetzen, es können jedoch auch in vorteilhafter Weise, Gemische verschiedener modifizierter und/oder unmodifizierter Schichtsilicate den erfindungsgemäßen Zusammensetzungen einzuverleiben.

Unter Schichtsilicaten, welche auch Phyllosilicate genannt werden, sind im Rahmen dieser Anmeldung Silicate und Alumosilicate zu verstehen, in welchen die Silicat- bzw. Aluminateinheiten über drei Si-O- oder Al-O- Bindungen untereinander verknüpft sind und eine gewellte Blatt- oder Schichtenstruktur ausbilden. Die vierte Si-O- bzw. Al-O- Valenz wird durch Kationen abgesättigt. Zwischen den einzelnen Schichten bestehen schwächere elektrostatische Wechselwirkungen, z.B. Wasserstoffbrückenbindungen. Das Schichtgefüge indessen ist weitgehend durch starke, kovalente Bindungen geprägt.

Die Stöchiometrie der Blattsilicate ist
(Si₂O₅²⁻) für reine Silicatstrukturen und
(AlₘSi²⁻ₘO₅(^{2+m})⁻) für Alumosilicate.
m ist eine Zahl größer als Null und kleiner als 2.

Liegen keine reinen Silicate sondern Alumosilicate vor, ist dem Umstande Rechnung zu tragen, daß jede durch Al³⁺ ersetzte Si⁴⁺ - Gruppe ein weiteres einfach geladenes Kation zur Ladungsneutralisierung erfordert.

Die Ladungsbilanz wird bevorzugt durch H⁺, Alkali- oder Erdalkalimetallionen ausgeglichen. Auch Aluminium als Gegenion ist bekannt und vorteilhaft. Im Gegensatz zu den Alumosilicaten werden diese Verbindungen Aluminiumsilicate genannt. Auch "Aluminiumalumosilicate", in welchen Aluminium sowohl im Silicatnetz, als auch als Gegenion vorliegt, sind bekannt und für die vorliegende Erfindung gegebenenfalls von Vorteil.

Schichtsilicate sind in der Literatur gut dokumentiert, z.B. im "Lehrbuch der Anorganischen Chemie", A.F. Hollemann, E. Wiberg und N. Wiberg, 91.-100. Aufl., Walter de Gruyter - Verlag 1985, passim, sowie "Lehrbuch der Anorganischen Chemie", H.Remy, 12. Aufl., Akademische Verlagsgesellschaft, Leipzig 1965, passim. Die Schichtenstruktur von Montmorillonit ist Römpps Chemie-Lexikon, Franckh'sche Verlagshandlung W. Keller & Co., Stuttgart, 8.Aufl., 1985, S. 2668 f., zu entnehmen.

Beispiele für Schichtsilicate sind:
- Montmorillonit: Na_{0,33}((Al_{1,67}Mg_{0,33})(OH)₂(Sᵢ₄O₁₀))
- oft vereinfacht:: Al₂O₃*4SiO₂*H₂O*nH₂O bzw. Al₂[(OH)₂/Si₄O₁₀] · n H₂O
- Kaolinit: Al₂(OH)₄(Si₂O₅)
- Ilit: (K,H₃O)_{y}(Mg₃(OH)₂(Si_{4-y}Al_{y}O₁₀))
- und: (K,H₃O)_{y}(Al₂(OH)₂(Si_{4-y}Al_{y}O₁₀))
mit y = 0,7 - 0,9
- Beidellit: (Ca,Na)_{0,3}(Al₂(OH)₂(Al_{0,5}Si_{3,5}O₁₀))
- Nontronit: Na_{0,33}(Fe₂(OH)₂(Al_{0,33}S_{i3,67}O₁₀))
- Saponit: (Ca,Na)_{0,33}((Mg,Fe)₃(OH)₂(Al_{0,33}Si_{3,67}O₁₀))
- Hectorit: Na_{0,33}((Mg,Li)₃(OH,F)₂(Si₄O₁₀))

Montmorillonit stellt das Hauptmineral der natürlich vorkommenden Bentonite dar.

Sehr vorteilhafte anorganische Gelbildner im Sinne der vorliegenden Erfindung sind Aluminiumsilikate wie die Montmorillonite (Bentonite, Hectorite sowie deren Derivate wie Quaternium-18 Bentonit, Quaternium-18 Hectorite, Stearalkonium Bentonite bzw. Stearalkonium Hectorite) oder aber Magnesium-Aluminium-Silikate (Veegum®-Typen) sowie Natrium-Magnesium-Silikate (Laponite®-Typen)

Montmorillonite stellen zu den dioktaedrischen Smektiten gehörende Tonmineralien dar und sind in Wasser quellende, aber nicht plastisch werdende Massen. Die Schichtpakete in der Dreischicht-Struktur der Montmorillonite können durch reversible Einlagerung von Wasser (in der 2-7fachen Menge) u. a. Substanzen wie z. B. Alkoholen, Glykolen, Pyridin, α-Picolin, Ammonium-Verbindungen, Hydroxy-Aluminosilicat-lonen usw. aufquellen.

Die oben angegebene chemische Formel ist nur angenähert; da M. ein großes lonenaustausch-Vermögen besitzt, kann Al gegen Mg, Fe²⁺, Fe³⁺, Zn, Pb (z. B. aus Schadstoffen in Abwässern ) Cr, auch Cu und andere ausgetauscht werden. Die daraus resultierende negative Ladung der Oktaeder-Schichten wird durch Kationen, insbesondere Na⁺ (Natrium-Montmorillonit) und Ca²⁺ (der Calcium-Montmorillonit ist nur sehr wenig quellfähig) in Zwischenschicht-Positionen ausgeglichen.

Im Sinne der vorliegenden Erfindung vorteilhafte synthetische Magnesiumsilikate bzw. Bentonite werden beispielsweise von Süd-Chemie unter der Handelsbezeichung Optigel® vertrieben.

Ein im Sinne der vorliegenden Erfindung vorteilhaftes Aluminiumsilikat wird beispielsweise von der R. T. Vanderbilt Comp., Inc., unter der Handelsbezeichnung Veegum® vertrieben. Die verschiedenen Veegum®-Typen, welche alle erfindungsgemäß vorteilhaft sind, zeichnen sich durch folgende Zusammensetzungen aus

| | (regular grade) | HV | K | HS | S-728 |
|---|---|---|---|---|---|
| SiO₂ | 55,5 | 56,9 | 64,7 | 69,0 | 65,3 |
| MgO | 13,0 | 13,0 | 5,4 | 2,9 | 3,3 |
| Al₂O₃ | 8,9 | 10,3 | 14,8 | 14,7 | 17,0 |
| Fe₂O₃ | 1,0 | 0,8 | 1,5 | 1,8 | 0,7 |
| CaO | 2,0 | 2,0 | 1,1 | 1,3 | 1,3 |
| Na₂O | 2,1 | 2,8 | 2,2 | 2,2 | 3,8 |
| K₂O | 1,3 | 1,3 | 1,9 | 0,4 | 0,2 |
| Veraschungsve rlust | 11,1 | 12,6 | 7,6 | 5,5 | 7,5 |

Diese Produkte quellen in Wasser unter Bildung viskoser Gele, welche alkalisch reagieren. Durch Organophilierung von Montmorillonit bzw. Bentoniten (Austausch der Zwischenschicht-Kationen gegen quaternäre Alkylammonium-lonen) entstehen Produkte (Bentone), die bevorzugt zur Dispergierung in organischen Lösemitteln und Ölen, Fetten, Salben, Farben, Lacken und in Waschmitteln eingesetzt werden.

Bentone® ist eine Handelsbezeichnung für verschiedene neutrale und chemisch inerte Geliermittel, die aus langkettigen, organischen Ammoniumsalzen und speziellen Montmorillonit-Sorten aufgebaut sind. Bentone quellen in organischen Medien und bringen diese zum Quellen. Die Gele sind in verdünnten Säuren und Alkalien beständig, bei längerer Berührung mit starken Säuren und Alkalien verlieren sie ihre Geliereigenschaften jedoch teilweise. Aufgrund ihres organophilen Charakters sind die Bentone nur schwer durch Wasser benetzbar.

Folgende Bentone® -Typen werden beispielsweise von der Gesellschaft Kronos Titan vertrieben: Bentone® 27, ein organisch modifiziertes Montmorillonit, Bentone® 34 (Dimethyldioctylammoniumbentonit), das nach US 2,531,427 hergestellt wird und wegen seiner lipophilen Gruppen besser im lipophilen Medium als in Wasser quillt, Bentone® 38, ein organisch modifiziertes Montmorillonit, ein cremefarbenes bis weißes Pulver, Bentone® LT, ein gereinigtes Tonmineral, Bentone® Gel MIO, ein organisch modifiziertes Montmorillonit, das in Mineralöl (SUS-71) feinst suspendiert angeboten wird (10 % Bentonit, 86,7 % Mineralöl und 3,3 % Netzmittel), Bentone® Gel IPM, ein organisch modifiziertes Bentonit, das in Isopropylmyristat suspendiert ist (10 % Bentonit, 86,7 % Isopropylmyristat, 3,3 % Netzmittel), Bentone® Gel CAO, ein organisch modifiziertes Montmorillonit, das in Ricinusöl aufgenommen ist (10 % Bentonit, 86,7 % Ricinusöl und 3,3 % Netzmittel), Bentone® Gel Lantrol, ein organisch modifiziertes Montmorillonit, das in Pastenform zur Weiterverarbeitung, insbesondere zur Herstellung kosmetischer Mittel bestimmt ist; 10 % Bentonit, 64,9 Lantrol (Wollwachsöl), 22,0 Isopropylmyristat, 3,0 Netzmittel und 0,1 p-Hydroxybenzoesäurepropylester, Bentone® Gel Lan I, eine 10 %ige Bentone® 27-Paste in einer Mischung aus Wollwachs USP und Isopropylpalmitat, Bentone® Gel Lan II, eine Bentonit-Paste in reinem, flüssigem Wollwachs, Bentone® Gel NV, eine 15 %ige Bentone® 27-Paste in Dibutylphthalat, Bentone® Gel OMS, eine Bentonit-Paste in Shellsol T. Bentone® Gel OMS 25, eine Bentonit Paste in Isoparaffinischen Kohlenwasserstoffen (Idopar® H), Bentone® Gel IPP, eine Bentonit-Paste in Isopropylpalmitat.

Die partikulären hydrophoben und/oder hydrophobisierten und/oder ölabsorbierenden Festkörpersubstanzen können beispielsweise vorteilhaft gewählt werden aus der Gruppe
- der modifizierten oder unmodifizierten Schichtsilikate.
- der modifizierten Kohlenhydratderivate wie Cellulose und Cellulosederivate, mikrocristalline Cellulose, Stärke und Stärke-Derivate (Distärkephosphat, Natrium- bzw. Aluminium-Stärkeoctenylsuccinat, Weizenstärke, Maisstärke (Amidon De Mais MST (Wackherr), Argo Brand Maisstärke (Corn Products), Pure-Dent (Grain Processing), Purity 21C (National Starch), Reisstärke (D.S.A. 7 (Agrana Stärke), Oryzapearl (Ichimaru Pharcos), Natriummaisstärkeoctenylsuccinat (C* EmCap - Instant 12639 (Cerestar USA)) Aluminium-Stärkeoctenylsuccinat (Covafluid AMD (Wackherr) Dry Flo-PC (National Starch) Dry Flo Pure (National Starch) Fluidamid DF 12 (Roquette))
- der anorganischen Füllstoffe (wie Talkum, Kaolin, Zeolithe, Bornitrid)
- der anorganischen Pigmente auf Basis von Metalloxiden und / oder anderen in Wasser schwerlöslichen bzw. unlöslichen Metallverbindungen (insb. Oxide des Titans, Zinks, Eisens, Mangans, Aluminium, Cers)
- der anorganischen Pigmente auf Basis von Silicumoxiden (wie insbesondere die Typen Aerosil-200, Aerosil 200 V).
- der Silikat-Derivate (wie Natrium Silicoaluminate, Magnesiumsilicate, Natriummagnesiumsilicate (Laponite-Typen), Magnesiumaluminiumsilikate (Sebumasse) oder Fluoro Magnesium Silicate (Submica-Typen), Calcium Aluminium Borsilicate). Bevorzugt ist hierbei insbesondere Silica Dimethyl Silylate (Aerosil R972).

Mikrokristalline Cellulose ist ein vorteilhaftes Festkörpersubstanzen im Sinne der vorliegenden Erfindung. Sie ist beispielsweise von der "FMC Corporation Food and Pharmaceutical Products" unter der Handelsbezeichnung Avicel® erhältlich. Ein besonders vorteilhaftes Produkt im Sinne der vorliegenden Erfindung ist der Typ Avicel® RC-591, bei dem es sich um modifizierte mikrokristalline Cellulose handelt, die sich zu 89% aus mikrokristalliner Cellulose und zu 11% aus Natrium Carboxymethyl Cellulose zusammensetzt. Weitere Handelsprodukte dieser Rohstoffklasse sind Avicel® RC/ CL, Avicel® CE-15, Avicel® 500.

Weitere erfindungsgemäß vorteilhafte ölabsorbiernde Festkörpersubstanzen sind microsphärische Partikel, die auf quervernetzten Polymethylmethacrylate (INCI: Crosslinked Methylmethacrylate) basieren. Diese werden von SEPPIC unter den Handelbezeichnungen Micropearl® M305, Micropearl® 201, Micropearl® M 310 und Micropearl® MHB vertrieben und zeichnen sich durch ein Ölaufnahmevermögen von 40-100 g / 100g aus.

Aerosile (fumed Silica) = durch thermische Zersetzung von Ethylsilicat gewonnenes Siliciumdioxid) sind hochdisperse Kieselsäuren mit häufig irregulärer Form, deren spezifische Oberfläche in der Regel sehr groß ist (200 - 400 m² / g) und abhängig vom Herstellverfahren gesteuert werden kann.

Erfindungsgemäß vorteilhaft zu verwendende Aerosile sind beispielsweise erhältlich unter den Handelsnamen: Aerosil® 130 (Degussa Hüls) Aerosil® 200 (Degussa Hüls) Aerosil 255 (Degussa Hüls) Aerosil® 300 (Degussa Hüls) Aerosil® 380 (Degussa Hüls) B-6C (Suzuki Yushi) CAB-O-SIL Fumed Silica (Cabot) CAB-O- SIL EH-5 (Cabot) CAB-O-SIL HS-5 (Cabot) CAB-O-SIL LM-130 (Cabot) CAB-O-SIL MS-55 (Cabot) CAB-O-SIL M-5 (Cabot) E-6C (Suzuki Yushi) Fossil Flour MBK (MBK) MSS-500 (Kobo) Neosil CT 11 (Crosfield Co.) Ronasphere (Rona/EM Industries) Silica, Anhydrous 31 (Whittaker, Clark & Daniels) Silica, Crystalline 216 (Whittaker, Clark & Daniels) Silotrat-1 (Vevy) Sorbosil AC33 (Crosfield Co.) Sorbosil AC 35 (Crosfield Co.) Sorbosil AC 37 (Crosfield Co.) Sorbosil AC 39 (Crosfield Co.) Sorbosil AC77 (Crosfield Co.) Sorbosil TC 15 (Crosfield Co.) Spherica (Ikeda) Spheriglass (Potters-Ballotini) Spheron L-1500 (Presperse) Spheron N-2000 (Presperse) Spheron P-1500 (Presperse) Wacker HDK H 30 (Wacker-Chemie) Wacker HDK N 20 (Wacker-Chemie) Wacker HDK P 100 H (Wacker Silicones) Wacker HDK N 20P (Wacker-Chemie) Wacker HDK N 25P (Wacker-Chemie) Wacker HDK S 13 (Wacker-Chemie) Wacker HDK T 30 (Wacker-Chemie) Wacker HDK V 15 (Wacker-Chemie) Wacker HDK V 15 P (Wacker-Chemie) Zelec Sil (DuPont)

Weiterhin ist vorteilhaft, solche SiO₂-Pigmente zu verwenden, bei welchen die freien OH Gruppen an der Teilchenoberfläche (ganz oder teilweise) organisch modifiziert worden sind. Man erhält z.B. durch die Addition von Dimethylsilyl-Gruppen Silica Dimethyl Silylate (z.B. Aerosil® R972 (Degussa Hüls) Aerosil® R974 (Degussa Hüls) CAB-O-SIL TS-610 (Cabot) CAB-O-SIL TS-720 (Cabot) Wacker HDK H15 (Wacker-Chemie) Wacker HDK H18 (Wacker-Chemie) Wacker HDK H20 (Wacker-Chemie)). Durch die Addition von Trimethylsily-Gruppen erhält man Silica Silylate (z.B. Aerosil R 812 (Degussa Hüls) CAB-O-SIL TS-530 (Cabot) Sipernat D 17 (Degussa Hüls) Wacker HDK H2000 (Wacker-Chemie)).

Polymethylsilsesquioxane werden beispielsweise unter den Handelsnamen Tospearl® 2000 B von GE Bayer Silikones, Tospearl 145A von Toshiba, AEC Silicone Resin Spheres von A & E Connock oder Wacker - Belsil PMS MK von der Wacker-Chemie angeboten.

Die erfindungsgemäßen kosmetischen und/oder dermatologischen Zubereitungen können wie üblich zusammengesetzt sein. Besonders vorteilhaft im Sinne der vorliegenden Erfindung sind Zubereitungen zur Pflege der Haut: sie können dem kosmetischen und/oder dermatologischen Lichtschutz, ferner zur Behandlung der Haut und/oder der Haare und als Schminkprodukt in der dekorativen Kosmetik dienen. Eine weitere vorteilhafte Ausführungsform der vorliegenden Erfindung besteht in After-Sun-Produkten.

Entsprechend ihrem Aufbau können kosmetische oder topische dermatologische Zusammensetzungen im Sinne der vorliegenden Erfindung, beispielsweise verwendet werden als Hautschutzcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Ebenso wie Emulsionen von flüssiger und fester Konsistenz als kosmetische Reinigungslotionen bzw. Reinigungscremes Verwendung finden, können auch die erfindungsgemäßen Zubereitungen "Reinigungsschäume" darstellen, welche beispielsweise zum Entfernen von Schminken und/oder Make-up oder als milder Waschschaum - ggf. auch für unreine Haut - verwendet werden können. Derartige Reinigungsschäume können vorteilhaft ferner als sogenannte "rinse off" Präparate angewendet werden, welche nach der Anwendung von der Haut abgespült werden

Die erfindungsgemäßen kosmetischen und/oder dermatologischen Zubereitungen können auch vorteilhaft in Form eines Schaums zur Pflege des Haars bzw. der Kopfhaut vorliegen, insbesondere eines Schaums zum Einlegen der Haare, eines Schaums, der beim Fönen der Haare verwendet wird, eines Frisier- und Behandlungsschaums.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Die kosmetischen und dermatologischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Bakterizide, Parfüme, Farbstoffe, Pigmente, die eine färbende Wirkung haben, anfeuchtende und/oder feuchthaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise Formaldehydabspalter (wie z. B. DMDM Hydantoin), lodopropylbutylcarbamate (z. B. die unter den Handelsbezeichnungen Koncyl-L, Koncyl-S und Konkaben LMB von der Fa. Lonza erhältlichen), Parabene, Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Carnosin und deren Derivate (z. B. Anserin), Carotinoide, Carotine (z. B. α-Carotin, β-Carotin, Lycopin) und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg - Ascorbylphosphat, Ascorbylacetat), Tocopherole und Derivate (z. B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, ZnSO₄) Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Eine erstaunliche Eigenschaft der erfindungsgemäße Zubereitungen ist, daß diese sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Erfindungsgemäß können die Wirkstoffe (eine oder mehrere Verbindungen) auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z. B. Hydrocortison-17-valerat; Vitamine der B- und D-Reihe, sehr günstig das Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die gamma-Linolensäure, Ölsäure, Eicosapentaënsäure, Docosahexaënsäure und deren Derivate, Chloramphenicol, Coffein, Prostaglandine, Thymol, Campher, Extrakte oder andere Produkte pflanzlicher und tierischer Herkunft, z. B. Nachtkerzenöl, Borretschöl oder Johannisbeerkernöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl, Eucerit® und Neocerit®.

Besonders vorteilhaft werden der oder die Wirkstoffe ferner gewählt aus der Gruppe der NO-Synthasehemmer, insbesondere wenn die erfindungsgemäßen Zubereitungen zur Behandlung und Prophylaxe der Symptome der intrinsischen und/oder extrinsischen Hautalterung sowie zur Behandlung und Prophylaxe der schädlichen Auswirkungen ultravioletter Strahlung auf die Haut dienen sollen.

Bevorzugter NO-Synthasehemmer ist das Nitroarginin.

Weiter vorteilhaft werden der oder die Wirkstoffe gewählt aus der Gruppe, welche Catechine und Gallensäureester von Catechinen und wäßrige bzw. organische Extrakte aus Pflanzen oder Pflanzenteilen umfaßt, die einen Gehalt an Catechinen oder Gallensäureestern von Catechinen aufweisen, wie beispielsweise den Blättern der Pflanzenfamilie Theaceae, insbesondere der Spezies Camellia sinensis (grüner Tee). Insbesondere vorteilhaft sind deren typische Inhaltsstoffe (wie z. B. Polyphenole bzw. Catechine, Coffein, Vitamine, Zucker, Mineralien, Aminosäuren, Lipide).

Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydroxyphenyl)-chroman-3,5,7-triol) darstellen. Auch Epicatechin ((2R,3R)-3,3',4',5,7-Flavanpentaol) ist ein vorteilhafter Wirkstoff im Sinne der vorliegenden Erfindung.

Vorteilhaft sind ferner pflanzliche Auszüge mit einem Gehalt an Catechinen, insbesondere Extrakte des grünen Tees, wie z. B. Extrakte aus Blättern der Pflanzen der Spezies Camellia spec., ganz besonders der Teesorten Camellia sinenis, C. assamica, C. taliensis bzw. C. irrawadiensis und Kreuzungen aus diesen mit beispielsweise Camellia japonica.

Bevorzugte Wirkstoffe sind ferner Polyphenole bzw. Catechine aus der Gruppe (-)-Catechin, (+)-Catechin, (-)-Catechingallat, (-)-Gallocatechingallat, (+)-Epicatechin, (-)-Epicatechin, (-)-Epicatechin Gallat, (-)-Epigallocatechin, (-)-Epigallocatechingallat.

Auch Flavon und seine Derivate (oft auch kollektiv "Flavone" genannt) sind vorteilhafte Wirkstoffe im Sinne der vorliegenden Erfindung. Sie sind durch folgende Grundstruktur gekennzeichnet (Substitutionspostitionen angegeben):

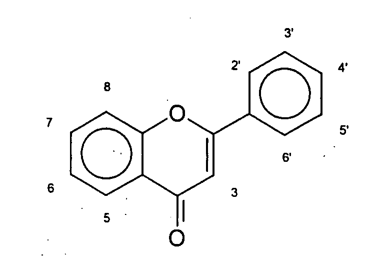

Einige der wichtigeren Flavone, welche auch bevorzugt in erfindungsgemäßen Zubereitungen eingesetz werden können, sind in der nachstehenden Tabelle aufgeführt:

| | OH-Substitutionspositionen | | | | | | | |
|---|---|---|---|---|---|---|---|---|
| | 3 | 5 | 7 | 8 | 2' | 3' | 4' | 5' |
| Flavon | - | - | - | - | - | - | - | - |
| Flavonol | + | - | - | - | - | - | - | - |
| Chrysin | - | + | + | - | - | - | - | - |
| Galangin | + | + | + | - | - | - | - | - |
| Apigenin | - | + | + | - | - | - | + | - |
| Fisetin | + | - | + | - | - | + | + | - |
| Luteolin | - | + | + | - | - | + | + | - |
| Kämpferol | + | + | + | - | - | - | + | - |
| Quercetin | + | + | + | - | - | + | + | - |
| Morin | + | + | + | - | + | - | + | - |
| Robinetin | + | - | + | - | - | + | + | + |
| Gossypetin | + | + | + | + | - | + | + | - |
| Myricetin | + | + | + | - | - | + | + | + |

In der Natur kommen Flavone in der Regel in glycosidierter Form vor.

Erfindungsgemäß werden die Flavonoide bevorzugt gewählt gewählt aus der Gruppe der Substanzen der generischen Strukturformel 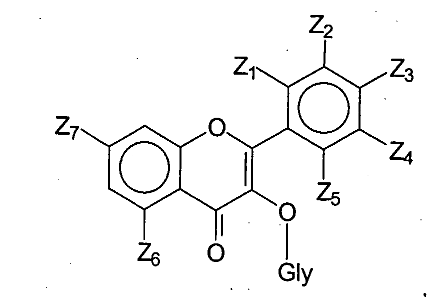 wobei Z₁ bis Z₇ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Erfindungsgemäß können die Flavonoide aber auch vorteilhaft gewählt werden aus der Gruppe der Substanzen der generischen Strukturformel 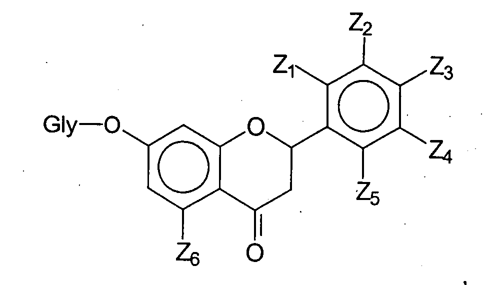 wobei Z₁ bis Z₆ unabhängig voneinander gewählt werden aus der Gruppe H, OH, Alkoxysowie Hydroxyalkoxy-, wobei die Alkoxy- bzw. Hydroxyalkoxygruppen verzweigt und unverzweigt sein und 1 bis 18 C-Atome aufweisen können, und wobei Gly gewählt wird aus der Gruppe der Mono- und Oligoglycosidreste.

Bevorzugt können solche Strukturen gewählt werden aus der Gruppe der Substanzen der generischen Strukturformel 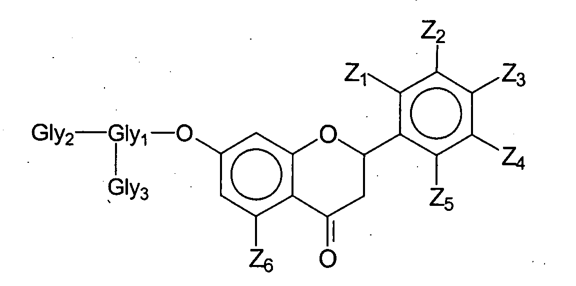 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Vorteilhaft werden Z₁ bis Z₅ unabhängig voneinander gewählt aus der Gruppe H, OH, Methoxy-, Ethoxy- sowie 2-Hydroxyethoxy-, und die Flavonglycoside haben die Struktur

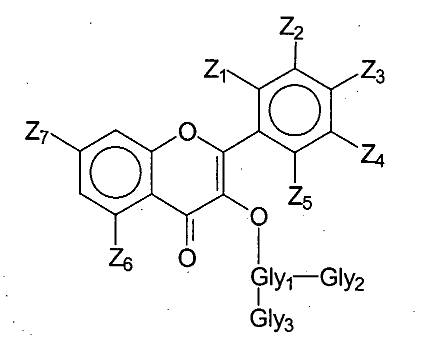

Besonders vorteilhaft werden die erfindungsgemäßen Flavonglycoside aus der Gruppe, welche durch die folgende Struktur wiedergegeben werden: 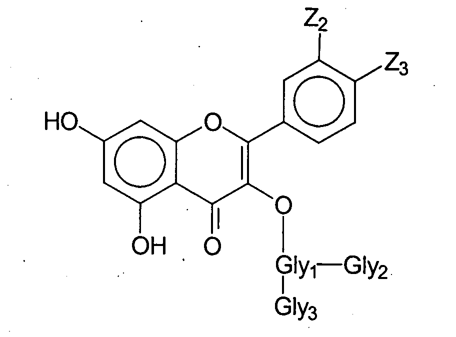 wobei Gly₁, Gly₂ und Gly₃ unabhängig voneinander Monoglycosidreste oder darstellen. Gly₂ bzw. Gly₃ können auch einzeln oder gemeinsam Absättigungen durch Wasserstoffatome darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Besonders vorteilhaft im Sinne der vorliegenden Erfindung ist, das oder die Flavonglycoside zu wählen aus der Gruppe α-Glucosylrutin, α-Glucosylmyricetin, α-Glucosylisoquercitrin, α-Glucosylisoquercetin und α-Glucosylquercitrin.

Erfindungsgemäß besonders bevorzugt ist α-Glucosylrutin.

Erfindungsgemäß vorteilhaft sind auch Naringin (Aurantiin, Naringenin-7-rhamnoglucosid), Hesperidin (3',5,7-Trihydroxy-4'-methoxyflavanon-7-rutinosid, Hesperidosid, Hesperetin-7-O-rutinosid). Rutin (3,3',4',5,7-Pentahydroxyflyvon-3-rutinosid, Quercetin-3-rutinosid, Sophorin, Birutan, Rutabion, Taurutin, Phytomelin, Melin), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Dihydrorobinetin (3,3',4',5',7-Pentahydroxyflavanon), Taxifolin (3,3',4',5,7-Pentahydroxyflavanon), Eriodictyol-7-glucosid (3',4',5,7-Tetrahydroxyflavanon-7-glucosid), Flavanomareïn (3',4',7,8-Tetrahydroxyflavanon-7-glucosid) und Isoquercetin (3,3',4',5,7-Pentahydroxyflavanon-3-(β-D-Glucopyranosid).

Vorteilhaft ist es auch, dem oder die Wirkstoffe aus der Gruppe der Ubichinone und Plastochinone zu wählen.

Ubichinone zeichnen sich durch die Strukturformel 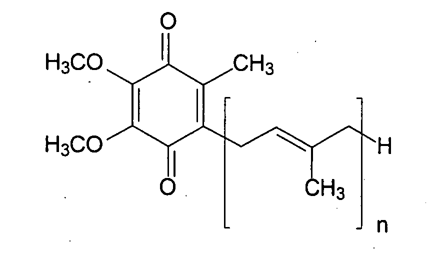 aus und stellen die am weitesten verbreiteten und damit am besten untersuchten Biochinone dar. Ubichinone werden je nach Zahl der in der Seitenkette verknüpften Isopren-Einheiten als Q-1, Q-2, Q-3 usw. oder nach Anzahl der C-Atome als U-5, U-10, U-15 usw. bezeichnet. Sie treten bevorzugt mit bestimmten Kettenlängen auf, z. B. in einigen Mikroorganismen und Hefen mit n=6. Bei den meisten Säugetieren einschließlich des Menschen überwiegt Q10.

Besonders vorteilhaft ist Coenzym Q10, welches durch folgende Strukturformel gekennzeichnet ist:

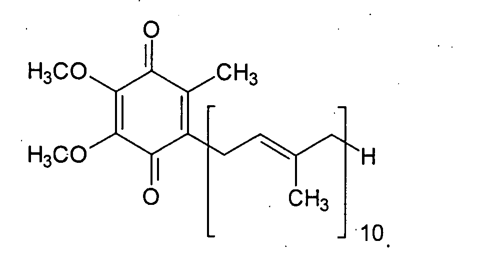

Plastochinone weisen die allgemeine Strukturformel 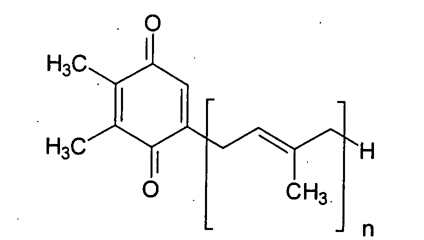 auf. Plastoschinone unterscheiden sich in der Anzahl n der Isopren-Reste und werden endsprechend bezeichnet, z. B. PQ-9 (n=9). Ferner existieren andere Plastochinone mit unterschiedlichen Substituenten am Chinon-Ring.

Auch Kreatin und/oder Kreatinderivate sind bevorzugte Wirkstoffe im Sinne der vorliegenden Erfindung. Kreatin zeichnet sich durch folgende Struktur aus:

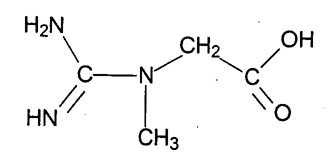

Bevorzugte Derivate sind Kreatinphosphat sowie Kreatinsulfat, Kreatinacetat, Kreatinascorbat und die an der Carboxylgruppe mit mono- oder polyfunktionalen Alkoholen veresterten Derivate.

Ein weiterer vorteilhafter Wirkstoff ist L-Carnitin [3-Hydroxy-4-(trimethylammonio)-buttersäurebetain]. Auch Acyl-Carnitine, welche gewählt aus der Gruppe der Substanzen der folgenden allgemeinen Strukturformel 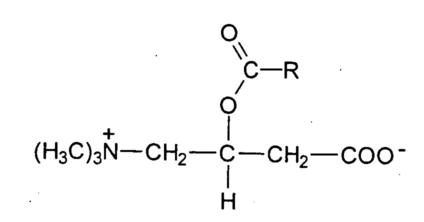 wobei R gewählt wird aus der Gruppe der verzweigten und unverzweigten Alkylreste mit bis zu 10 Kohlenstoffatomen sind vorteilhafte Wirkstoffe im Sinne der vorliegenden Erfindung. Bevorzugt sind Propionylcarnitin und insbesondere Acetylcarnitin. Beide Entantiomere (D- und L-Form) sind vorteilhaft im Sinne der vorliegenden Erfindung zu verwenden. Es kann auch von Vorteil sein, beliebige Enantiomerengemische, beispielsweise ein Racemat aus D- und L-Form, zu verwenden.

Weitere vorteilhafte Wirkstoffe sind Sericosid, Pyridoxol, Vitamin K, Biotin und Aromastoffe.

Die Liste der genannten Wirkstoffe bzw. Wirkstoffkombinationen, die in den erfindungsgemäßen Zubereitungen verwendet werden können, soll selbstverständlich nicht limitierend sein. Die Wirkstoffe können einzelnen oder in beliebigen Kombinationen miteinander verwendet werden.

Hautalterung wird z. B. durch endogene, genetisch determinierte Faktoren verursacht. In Epidermis und Dermis kommt es alterungsbedingt z. B. zu folgenden Strukturschäden und Funktionsstörungen, die auch unter den Begriff "Senile Xerosis" fallen können:
a) Trockenheit, Rauhigkeit und Ausbildung von (Trockenheits-) Fältchen,
b) Juckreiz und
c) verminderte Rückfettung durch Talgdrüsen (z. B. nach dem Waschen).
   Exogene Faktoren, wie UV-Licht und chemische Noxen, können kumulativ wirksam sein und z. B. die endogenen Alterungsprozesse beschleunigen bzw. sie ergänzen. In Epidermis und Dermis kommt es insbesondere durch exogene Faktoren z. B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z. B. Altersflecken) und
g) vergrößerte Anfälligkeit gegenüber mechanischem Stress (z. B. Rissigkeit).

Erstaunlicherweise können ausgewählte erfindungsgemäße Rezepturen auch eine Antifaltenwirkung aufweisen bzw. die Wirkung bekannter Antifaltenwirkstoffe erheblich steigern. Dementsprechend eignen sich Formulierungen im Sinne der vorliegenden Erfindung insbesondere vorteilhaft zur Prophylaxe und Behandlung kosmetischer oder dermatologischer Hautveränderungen, wie sie z. B. bei der Hautalterung auftreten. Weiterhin vorteilhaft eignen sie sich gegen das Erscheinungsbild der trockenen bzw. rauhen Haut.

In einer besonderen Ausführungsform betrifft die vorliegende Erfindung daher Produkte zur Pflege der auf natürliche Weise gealterten Haut, sowie zur Behandlung der Folgeschäden der Lichtalterung, insbesondere der unter a) bis g) aufgeführten Phänomene.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, Polymere, Schaumstabilisatoren, Elektrolyte sowie Moisturizer.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Die erfindungsgemäßen kosmetischen und dermatologischen Zubereitungen können Farbstoffe und/oder Farbpigmente enthalten, insbesondere wenn sie in Form von dekorativen Kosmetika vorliegen. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem *Rowe Colour Index, 3. Auflage, Society of Dyers and Colourists, Bradford, England, 1971* entnommen.

| **Chemische oder sonstige Bezeichnung** | **CIN** | **Farbe** |
|---|---|---|
| Pigment Green | 10006 | grün |
| Acid Green 1 | 10020 | grün |
| 2,4-Dinitrohydroxynaphthalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-benzol-5-sulfosäure | 13015 | gelb |
| 2,4-Dihydroxy-azobenzol-4'-sulfosäure | 14270 | orange |
| 2-(2,4-Dimethylphenylazo-5-sulfosäure)-1-hydroxynaphthalin-4-sulfosäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxy-naphthalin | 15525 | rot |
| 1-(3-Methyl-phenylazo-4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1 -(2-Sulfo-4-methyl-5-chlor-1-phenylazo)-2-hydroxy-naphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1 -(4-Sulfo-1 -naphthylazo)-2-naphthol-3,6,8-trisulfosäure | 16290 | rot |
| 8-Amino-2 -phenylazo- 1 -naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-pyrazolon-3-carbonsäure | 19140 | gelb |
| Pigment Yellow 16 | 20040 | gelb |
| 2,6-(4'-Sulfo-2", 4"-dimethyl)-bis-phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"-Sulfo-1"-phenylazo)-7'-sulfo-1'-naphthylazo]-1-hydroxy-7-aminonaphthalin-3,6-disulfosäure | 27755 | schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |
| Food Yellow | 40800 | orange |
| trans-β-Apo-8'-Carotinaldehyd (C₃₀) | 40820 | orange |
| trans-Apo-8'-Carotinsäure (C₃₀)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenyl-carbinol | 42051 | blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino)-phenyl-(2-sulfophenyl)-methylen-(N-ethyl-N-p-sulfo-benzyl)Δ^{2,5}-cyclohexadienimin | 42090 | blau |
| Acid Green 9 | 42100 | grün |
| Diethyl-di-sulfobenzyl-di-4-amino-2-chlor-di-2-methyl-fuchsonimmonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethylN-m-sulfobenzyl-fuchsonimmonium | 42735 | blau |
| 4'-(N-Dimethyl)-amino-4"-(N-phenyl)-aminonaphtho-N-dimethylfuchsonimmonium | 44045 | blau |
| 2-Hydroxy-3,6-disulfo-4,4'-bis-dimethylaminonaphthofuchsonimmonium | 44090 | grün |
| Acid Red 52 | 45100 | rot |
| 3-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid Violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidino)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha-, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Mangananimoniumdiphosphat | 77742 | violett |
| Manganphosphat; Mn₃(PO₄)₂ · 7 H20 | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalze, Anthocyane | | rot |
| Aluminium-, Zink-, Magnesium- und Calciumstearat | | weiß |
| Bromthymolblau | | blau |
| Bromkresolgrün | | grün |
| Acid Red 195 | | rot |

Sofern die erfindungsgemäßen Formulierungen in Form von Produkten vorliegen, welche im Gesicht angewendet werden, ist es günstig, als Farbstoff eine oder mehrere Substanzen aus der folgenden Gruppe zu wählen: 2,4-Dihydroxyazobenzol, 1-(2'-Chlor-4'-nitro-1'phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Ferner vorteilhaft sind öllösliche Naturfarbstoffe, wie z. B. Paprikaextrakte, β-Carotin oder Cochenille.

Vorteilhaft im Sinne der vorliegenden Erfindung sind ferner Formulierungen mit einem Gehalt an Perlglanzpigmenten. Bevorzugt sind insbesondere die im folgenden aufgelisteten Arten von Perlglanzpigmenten:
1. Natürliche Perlglanzpigmente, wie z. B.
   ■ "Fischsilber" (Guanin/Hypoxanthln-Mlschkrlstalle aus Fischschuppen) und
   ■ "Perlmutt" (vermahlene Muschelschalen)
2. Monokristalline Perlglanzpigmente wie z. B. Bismuthoxychlorid (BiOCI)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| **Gruppe** | **Belegung / Schichtdicke** | **Farbe** |
|---|---|---|
| **Silberweiße Perlglanzpigmente** | TiO₂: 40 - 60 nm | silber |
| **Interferenzpigmente** | TiO₂: 60 - 80 nm | gelb |
| | TiO₂: 80 - 100 nm | rot |
| | TiO₂: 100 - 140 nm | blau |
| | TiO₂: 120 - 160 nm | grün |
| **Farbglanzpigmente** | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| **Kombinationspigmente** | TiO₂/Fe₂O₃ | Goldtöne |
| | TiO₂ / Cr₂O₃ | grün |
| | TiO₂ / Berliner Blau . | tiefblau |
| | TiO₂ / Carmin | rot |

Besonders bevorzugt sind z.B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO₂ und Fe₂O₃ beschichtete SiO₂-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Eisenperlglanzpigmente, welche ohne die Verwendung von Glimmer hergestellt werden. Solche Pigmente sind z. B. unter dem Handelsnamen Sicopearl Kupfer 1000 bei der Firma BASF erhältlich.

Besonders vorteilhaft sind ferner auch Effektpigmente, welche unter der Handelsbezeichnung Metasomes Standard / Glitter in verschiedenen Farben (yello, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (Cl) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise neben einer oder mehreren erfindungsgemäßen UV-Filtersubstanzen zusätzlich mindestens eine weitere UV-A- und/oder UV-B-Filtersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden.

Solche Pigmente können im Sinne der vorliegenden Erfindung vorteilhaft oberflächlich behandelt ("gecoatet") sein, wobei beispielsweise ein amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophoben Schicht versehen werden.

Erfindungsgemäß vorteilhaft sind z. B. Titandioxidpigmente, die mit Octylsilanol beschichtet sind. Geeignete Titandioxidpartikel sind unter der Handelsbezeichnung T805 bei der Firma Degussa erhältlich. Besonders vorteilhaft sind ferner mit Aluminiumstearat beschichtete TiO₂-Pigmente, z. B. die unter der Handelsbezeichnung MT 100 T bei der Firma TAYCA erhältlichen.

Eine weitere vorteilhafte Beschichtung der anorganische Pigmente besteht aus Dimethylpolysiloxan (auch: Dimethicon), einem Gemisch vollmethylierter, linearer Siloxanpolymere, die endständig mit Trimethylsiloxy-Einheiten blockiert sind. Besonders vorteilhaft im Sinne der vorliegenden Erfindung sind Zinkoxid-Pigmente, die auf diese Weise beschichtet werden.

Vorteilhaft ist ferner eine Beschichtung der anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhaft sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches durch die chemische Strukturformel 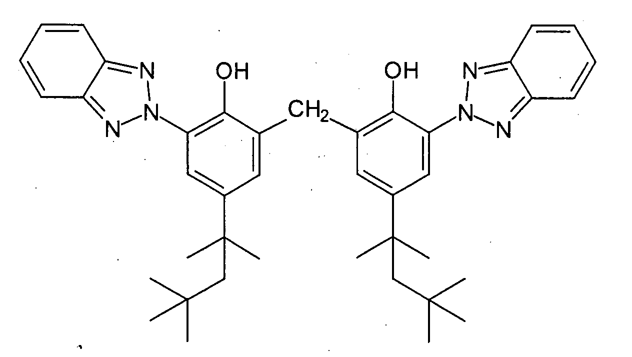 gekennzeichnet ist und unter der Handelsbezeichnung Tinosorb® M bei der CIBA-Chemikalien GmbH erhältlich ist.

Vorteilhaft enthalten erfindungsgemäße Zubereitungen Substanzen, die UV-Strahlung im UV-A- und/oder UV-B-Bereich absorbieren, wobei die Gesamtmenge der Filtersubstanzen z. B. 0,1 Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure 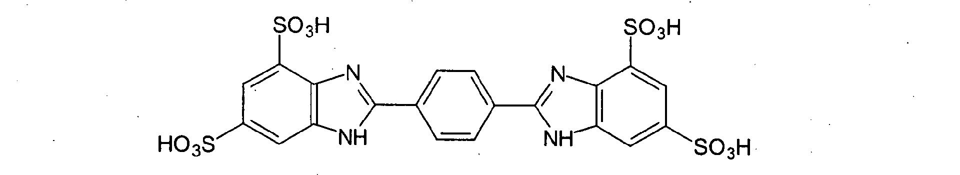 und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz 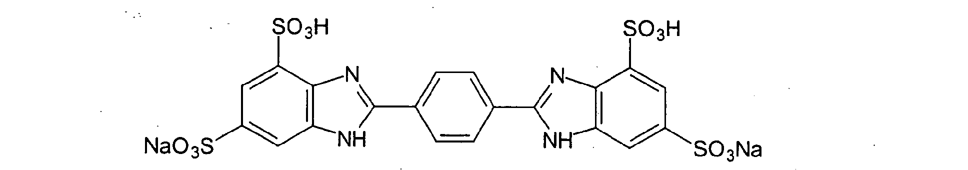 mit der INCI-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

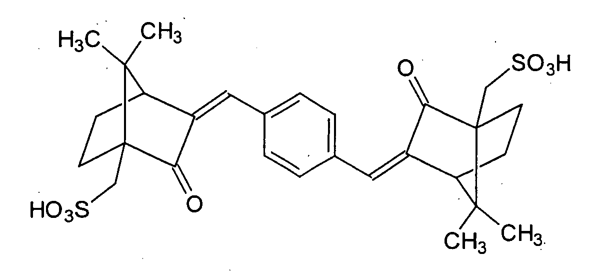

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur: 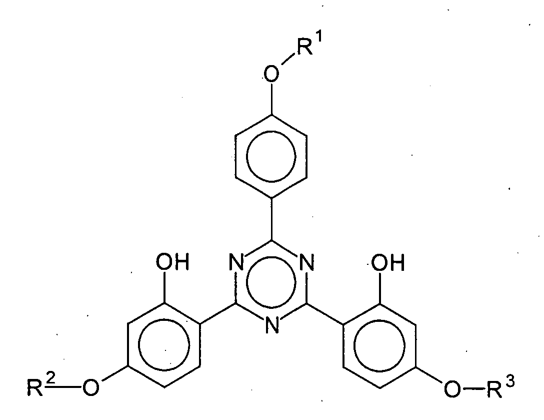 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydrpxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist.

Besonders vorteilhafte Zubereitungen im Sinne der vorliegenden Erfindung, die sich durch einen hohen bzw. sehr hohen UV-A-Schutz auszeichnen, enthalten bevorzugt mehrere UV-A- und/oder Breitbandfilter, insbesondere Dibenzoylmethanderivate [beispielsweise das 4-(tert.-Butyl)-4'-methoxydibenzoylmethan], Benzotriazolderivate [beispielsweise das 2,2'-Methylen-bis-(6-(2H-benzotriazol-2-yl)-4-(1,1,3,3-tetramethylbutyl)-phenol)], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Auch andere UV-Filtersubstanzen, welche das Strukturmotiv 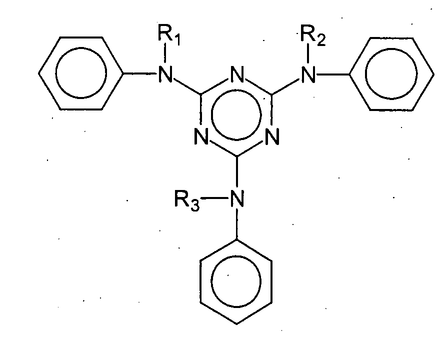 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 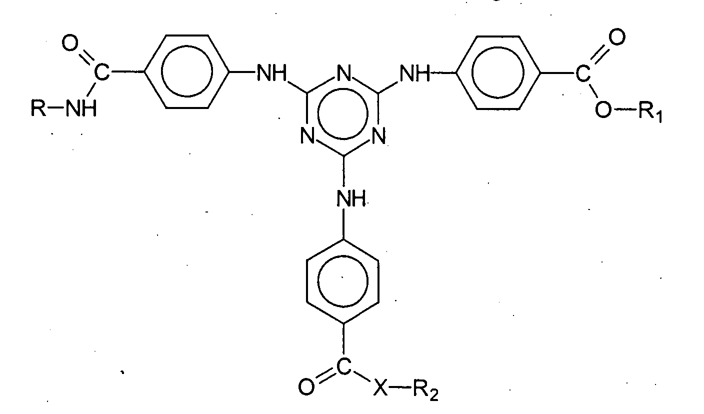 wiedergegeben wird, wobei
- R: einen verzweigten oder unverzweigten C₁-C₁₈-Alkylrest, einen C₅-C₁₂-Cycloalkyl-rest, gegebenenfalls substituiert mit einer oder mehreren C₁-C₄- Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkyl-rest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 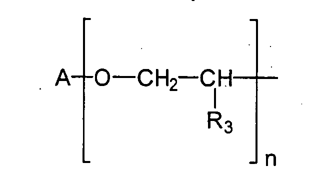 bedeutet, in welcher
A einen verzweigten oder unverzweigten C₁-C₁₈-Alkylrest, einen C₅-C₁₂-Cyclo-alkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
- R2: einen verzweigten oder unverzweigten C₁-C₁₈-Alkylrest, einen C₅-C₁₂-Cycloalkyl-rest, gegebenenfalls substituiert mit einer oder mehreren C₁-C₄- Alkylgruppen, darstellt, wenn X die NH-Gruppe darstellt, und einen verzweigten oder unverzweigten C₁-C₁₈-Alkylrest, einen C₅-C₁₂-Cycloalkyl-rest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 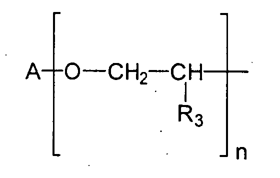 bedeutet, in welcher
A einen verzweigten oder unverzweigten C₁-C₁₈-Alkylrest, einen C₅-C₁₂-Cyclo-alkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel 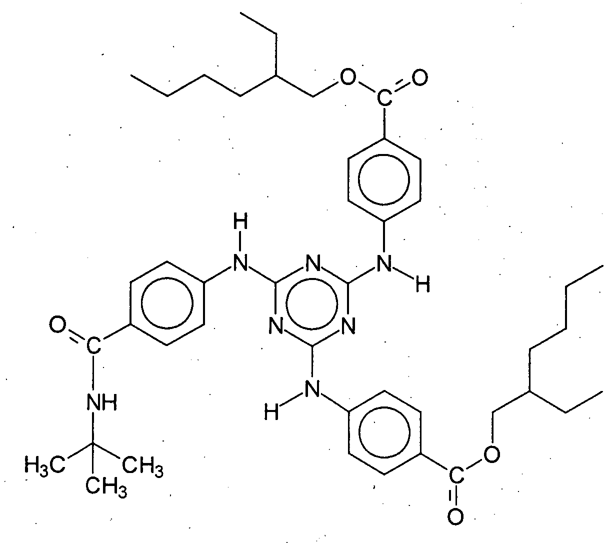 wiedergegeben wird, welches im Folgenden auch als Dioctylbutylamidotriazon (INCI: Dioctylbutamidotriazone) bezeichnet wird und unter der Handelsbezeichnung UVASORB HEB bei Sigma 3V erhältlich ist.

Vorteilhaft im Sinne der vorliegenden Erfindung ist auch ein symmetrisch substituiertes s-Triazin, das 4,4',4"-(1,3,5-Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel 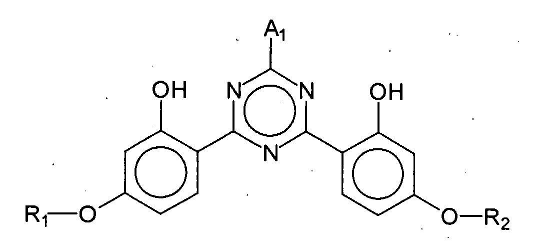 wiedergegeben wird, wobei R₁, R₂ und A₁ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxypropyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methylpropyloxy)-2-hydroxy)-phenyl)-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches durch die chemische Strukturformel 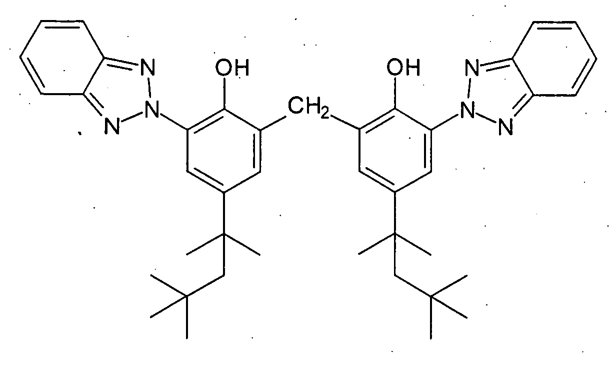 gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2Hbenzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches durch die chemische Strukturformel 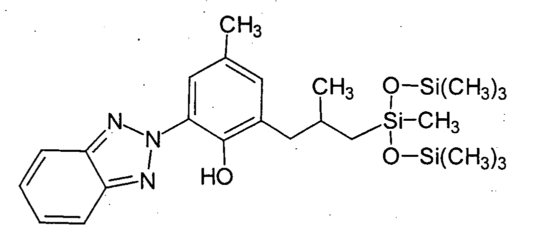 gekennzeichnet ist.

Die UV-B- und/oder Breitband-Filter können öllöslich oder wasserlöslich sein. Vorteilhafte öllösliche UV-B- und/oder Breitband-Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ 2,4,6-Trianilino-(p-carbo-2'-ethyl-1'-hexyloxy)-1,3,5-triazin;
■ Ester der Benzalmalonsäure, vorzugsweise 4-Methoxybenzalmalonsäuredi(2-ethylhexyl)ester;
■ Ester der Zimtsäure, vorzugsweise 4-Methoxyzimtsäure(2-ethylhexyl)ester, 4-Methoxyzimtsäureisopentylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Vorteilhafte wasserlösliche UV-B- und/oder Breitband-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist und sich durch folgende Struktur auszeichnet: 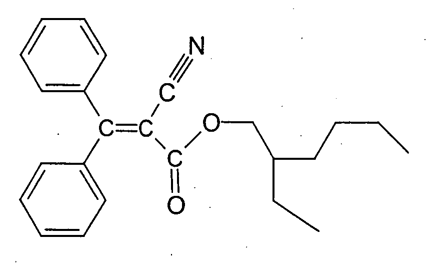 Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-Aund/oder UV-B-Filter in kosmetische oder dermatologische Zubereitungen einzuarbeiten, beispielsweise bestimmte Salicylsäurederivate wie 4-lsopropylbenzylsalicylat, 2-Ethylhexylsalicylat (= Octylsalicylat), Homomenthylsalicylat.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiel 1 (schaumförmige O/W-Creme):

| Emulsion I | Gew.-% | Vol.-% |
|---|---|---|
| Stearinsäure | 5,00 | |
| Cetylalkohol | 5,50 | |
| PEG-30 Stearat | 1,00 | |
| Cyclomethicon | 6,00 | |
| Isoeikosan | 1,00 | |
| Polydecen | 6,00 | |
| Magnesiumsilikat | 0,10 | |
| Carrageen | 0,10 | |
| Hydroxypropyldistärkephosphat | 1,00 | |
| Structure® XL | | |
| Aluminium Särke Octenylsuccinat | 0,20 | |
| Citronensäure | 0,10 | |
| Glycerin | 3,00 | |
| Natriumhydroxid | q.s. | |
| Konservierung | q.s. | |
| Parfum | q.s. | |
| Wasser, demineralisiert | ad 100,00 | |
| pH-Wert eingestellt auf 6,0 -7,5 | | |
| Emulsion I | | 60 |
| Gas (Luft) | | 40 |

Vordispergierung des anorganischen Gelbildners und Quellung der Hydrokolloide (u.a. des quervernetzten Stärkederivates) unter Rühren in der Wasserphase. Vereinigung der auf 75 °C aufgeheizten Fettphase mit der auf 70 °C aufgeheizten Wasserphase. Homogenisierung mittels einer Zahnkranzdispergiermaschine (Rotor-Stator-Prinzip) bei 65 °C. 45 min Rühren unter Begasung mit Luft bei 0.7bar und Kühlung. Zugabe des Aluminium Stärke Octenylsuccinates bei 35°C. Zugabe der Additive bei 30 °C (Parfüm, Wirkstoffe). Homogenisierung mittels einer Zahnkranzdispergiermaschine (Rotor-Stator-Prinzip) bei 27 °C.

### Beispiel 2 (schaumförmige O/W-Lotion):

| Emulsion II | Gew.-% | Vol.-% |
|---|---|---|
| Stearinsäure | 2,00 | |
| Myristylalkohol | 1,50 | |
| Cetylstearylalkohol | 0,50 | |
| PEG-100 Stearat | 3,00 | |
| Mineralöl | 5,00 | |
| Hydriertes Polyisobuten | 15,0 | |
| Magnesium-Aluminium-Silikat | 0,20 | |
| Hydroxypropyldistärkephosphat | 2,00 | |
| Structure® XL | | |
| Talkum | 1,25 | |
| Glycerin | 5,00 | |
| Natriumhydroxid | q.s. | |
| Konservierung | q.s. | |
| Parfum | q.s. | |
| Wasser,demineralisiert | ad 100,00 | |
| pH-Wert eingestellt auf 5,0-6,5 | | |
| Emulsion II | | 50 |
| Gas (Sauerstoff) | | 50 |

Vordispergierung des anorganischen Gelbildners und Quellung des Hydrokolloides (quervernetztes Stärkederivate) unter Rühren in der Wasserphase. Vereinigung der auf 80 °C aufgeheizten Fettphase mit der auf 72 °C aufgeheizten Wasserphase. Zugabe der partikulären hydrophoben, hydrophobisierten Festkörpersubstanzen unter Rühren. Homogenisierung mittels einer Zahnkranzdispergiermaschine (Rotor-Stator-Prinzip) bei 65 °C. 45 min Rühren unter Begasung mit Sauerstoff bei 1.2 bar und Kühlung. Zugabe der Additive bei 30 °C (Parfüm). Homogenisierung mittels einer Zahnkranzdispergiermaschine (Rotor-Stator-Prinzip) bei 30 °C.

### Beispiel 3 (schaumförmige O/W-Lotion):

| Emulsion III | Gew.-% | Vol.-% |
|---|---|---|
| Stearinsäure | 3,00 | |
| Cetylalkohol | 8,50 | |
| PEG-20 Stearat | 8,50 | |
| C₁₂₋₁₅ Alkyl Benzoate | 5,00 | |
| Isohexadecan | 2,00 | |
| Hydroxypropyldistärkephosphat | 7,00 | |
| Structure® XL | | |
| Bornitrid | 1,0 | |
| Glycerin | 10,0 | |
| Parfum, Konservierung, Farbstoffe | q.s. | |
| Natriumhydroxid | q.s. | |
| Wasser | ad 100 | |
| pH-Wert eingestellt auf 6,2 - 7,0 | | |
| Emulsion III | | 68 |
| Gas (Lachgas) | | 32 |

Quellung des Hydrokolloides (quervernetztes Stärkederivat) unter Rühren in der Wasserphase. Vereinigung der auf 80 °C aufgeheizten Fettphase mit der auf 75 °C aufgeheizten Wasserphase. Zugabe der partikulären hydrophoben, hydrophobisierten Festkörpersubstanzen (Bornitrid) unter Rühren. Homogenisierung mittels einer Zahnkranzdispergiermaschine (Rotor-Stator-Prinzip) bei 65 °C. 45 min Rühren unter Begasung mit Lachgas bei 0.7bar und Kühlung. Zugabe der Additive bei 30 °C (Parfüm, Wirkstoffe). Homogenisierung mittels einer Zahnkranzdispergiermaschine (Rotor-Stator-Prinzip) bei 25 °C.

### Beispiel 4 (schaumförmige O/W-Sonnenschutz-Creme):

| Emulsion IV | Gew.-% | Vol.-% |
|---|---|---|
| Palmitinsäure | 1,00 | |
| Cetylalkohol | 2,00 | |
| PEG-40 Stearat | 5,50 | |
| Cyclomethicon | 4,50 | |
| Dimethicon | 0,50 | |
| Dicaprylyl Ether | 2,00 | |
| Mineralöl | 4,50 | |
| Hectorit | 0,10 | |
| Polyacrylsäure | 0,10 | |
| Hydroxypropyldistärkephosphat | 5,00 | |
| Structure® XL | | |
| Kaolin | 1,25 | |
| Glycerin | 3,00 | |
| Octylmethoxycinnamat | 4,00 | |
| Butylmethoxydibenzoylmethan | 3,00 | |
| Ethylhexyl Triazon | 3,00 | |
| Glimmer | 1,00 | |
| Eisenoxid | 1,00 | |
| Titandioxid | 4,50 | |
| Vitamin A Palmitat | 0,10 | |
| Parfum, Konservierung | q.s. | |
| Natriumhydroxid | q.s. | |
| Wasser | ad 100 | |
| pH-Wert eingestellt auf 5,0 - 6,4 | | |
| Emulsion IV | | 43 |
| Gas (Argon) | | 57 |

Vordispergierung des anorganischen Gelbildners und Quellung der Hydrokolloide (u.a. des quervernetzten Stärkederivates) unter Rühren in der Wasserphase. Vereinigung der auf 80 °C aufgeheizten Fettphase mit der auf 72 °C aufgeheizten Wasserphase. Zugabe der partikulären hydrophoben, hydrophobisierten Festkörpersubstanzen unter Rühren. Homogenisierung mittels einer Zahnkranzdispergiermaschine (Rotor-Stator-Prinzip) bei 65 °C. 45 min Rühren unter Begasung mit Argon bei 1.2 bar und Kühlung. Zugabe der Additive bei 30 °C (Parfüm). Homogenisierung mittels einer Zahnkranzdispergiermaschine (Rotor-Stator-Prinzip) bei 30 °C.

### Beispiel 5 (schäumbare O/W-Creme):

| Emulsion V | Gew.-% | Vol.-% |
|---|---|---|
| Stearinsäure | 4.00 | |
| Cetylalkohol | 2.00 | |
| PEG-30 Stearat | 2.00 | |
| Sorbitan Monostearat | 1.50 | |
| Cyclomethicon | 5.00 | |
| Hydriertes Polyisobuten | 5.00 | |
| Quaternium-18-Hectorit | 0,10 | |
| Carrageen | 0,10 | |
| Xanthan Gummi | 0,05 | |
| Polyacrylsäure | 0,10 | |
| Hydroxypropyldistärkephosphat | 0,50 | |
| Structure® XL | | |
| SiliKa Dimethyl Silylat | 0,60 | |
| Glycerin | 3,00 | |
| Vitamin A Palmitat | 0,20 | |
| Vitamin-E-Acetat | 1,00 | |
| BHT | 0.02 | |
| Disodium EDTA | 0.10 | |
| Parfum, Konservierung, Farbstoffe | q.s. | |
| Kaliumhydroxid | q.s | |
| Wasser | ad 100 | |
| pH-Wert eingestellt auf 5,3 - 6,7 | | |
| Emulsion V | | 37 |
| Gas (Stickstoff) | | 63 |

Vordispergierung des anorganischen Gelbildners und Quellung der Hydrokolloide (u.a. des quervernetzten Stärkederivates) unter Rühren in der Wasserphase. Vereinigung der auf 80 °C aufgeheizten Fettphase mit der auf 72 °C aufgeheizten Wasserphase. Zugabe der partikulären hydrophoben, hydrophobisierten Festkörpersubstanzen unter Rühren.

Homogenisierung mittels einer Zahnkranzdispergiermaschine (Rotor-Stator-Prinzip) bei 65 °C. 45 min Rühren unter Begasung mit Stickstoff bei 1.2 bar und Kühlung. Zugabe der Additive bei 30 °C (Parfüm). Homogenisierung mittels einer Zahnkranzdispergiermaschine (Rotor-Stator-Prinzip) bei 30 °C.

### Beispiel 6 (schäumbare O/W-Lotion):

| Emulsion VI | Gew.-% | Vol.-% |
|---|---|---|
| Stearinsäure | 4.00 | |
| Cetylstearylalkohol | 1.00 | |
| PEG-100 Stearat | 1.00 | |
| Dimethicon | 0.50 | |
| Mineralöl | 6.50 | |
| Magnesium-Aluminium-Silikat | 0.10 | |
| Cellulosegummi | 0.10 | |
| Polyacrylsäure | 0,10 | |
| Hydroxypropyldistärkephosphat | 6,50 | |
| Structure® XL | | |
| Silizumoxid | 0,50 | |
| Glycerin | 3,00 | |
| Vitamin-E-Acetat | 2,00 | |
| Parfum, Konservierung, Farbstoffe | q.s. | |
| Kaliumhydroxid | q.s | |
| pH-Wert eingestellt auf 6,0 - 7,5 | | |
| Emulsion VI | | 64 |
| Gas (Helium) | | 36 |

Vordispergierung des anorganischen Gelbildners und Quellung der Hydrokolloide (u.a. des quervernetzten Stärkederivates) unter Rühren in der Wasserphase. Vereinigung der auf 80 °C aufgeheizten Fettphase mit der auf 72 °C aufgeheizten Wasserphase. Zugabe der partikulären hydrophoben, hydrophobisierten Festkörpersubstanzen unter Rühren. Homogenisierung mittels einer Zahnkranzdispergiermaschine (Rotor-Stator-Prinzip) bei 65 °C. 45 min Rühren unter Begasung mit Helium bei 1.2 bar und Kühlung. Zugabe der Additive bei 30 °C (Parfüm). Homogenisierung mittels einer Zahnkranzdispergiermaschine (Rotor-Stator-Prinzip) bei 30 °C.

### Beispiel 7 (schäumbare O/W-Sonnenschutz-Creme):

| Emulsion VII | Gew.-% | Vol.-% |
|---|---|---|
| Stearinsäure | 1,00 | |
| Cetylstearylalkohol | 4,00 | |
| Myristylalkohol | 1,00 | |
| PEG-20 Stearat | 1,00 | |
| Dimethicon | 0,50 | |
| Caprylsäure/Caprinsäuretriglycerid | 2,00 | |
| Octyl Isostearat | 5,00 | |
| Mineralöl | 15,0 | |
| Magnesiumsilikat | 0,10 | |
| Alkylacrylat Cosspolymer | 0,10 | |
| Hydroxypropyldistärkephosphat | 4,75 | |
| Structure® XL | | |
| Zeolith | 0,25 | |
| Glycerin | 5,50 | |
| Octylmethoxycinnamat | 5,00 | |
| Butylmethoxydibenzoylmethan | 3,00 | |
| Ethylhexyl Triazon | 4,00 | |
| BHT | 0,02 | |
| Disodium EDTA | 0,10 | |
| Parfum, Konservierung, Farbstoffe | q.s. | |
| Natriumhydroxid | q.s. | |
| Wasser | ad 100 | |
| pH-Wert eingestellt auf 6,0 - 7,5 | | |
| Emulsion VII | | 65 |
| Gas (Kohlendioxid) | | 35 |

Vordispergierung des anorganischen Gelbildners und Quellung der Hydrokolloide (u.a. des quervernetzten Stärkederivates) unter Rühren in der Wasserphase. Vereinigung der auf 80 °C aufgeheizten Fettphase mit der auf 72 °C aufgeheizten Wasserphase. Zugabe der partikulären hydrophoben, hydrophobisierten Festkörpersubstanzen unter Rühren. Homogenisierung mittels einer Zahnkranzdispergiermaschine (Rotor-Stator-Prinzip) bei 65 °C. 45 min Rühren unter Begasung mit Kohlendioxid bei 1.2 bar und Kühlung. Zugabe der Additive bei 30 °C (Parfüm). Homogenisierung mittels einer Zahnkranzdispergiermaschine (Rotor-Stator-Prinzip) bei 30 °C.

### Beispiel 8 (schaumförmige O/W-Lotion):

| Emulsion VI | Gew.-% | Vol.-% |
|---|---|---|
| Stearinsäure | 4,00 | |
| Cetylstearylalkohol | 1,00 | |
| PEG-100-Stearat | 1,00 | |
| Hydroxypropyldistärkephosphat | 1,50 | |
| Structure® XL | | |
| Paraffinöl | 6,50 | |
| Cyclomethcione | 2,00 | |
| Dimethicon | 0,50 | |
| Vitamin-E-Acetat | 2,00 | |
| Glycerin | 3,00 | |
| Dimethicone / Vinyl Dimethicone Cross- | 1,00 | |
| polymer | | |
| Magnesiumaluminiumsilikat | 0,50 | |
| Talkum | 0,50 | |
| PEG-180/Laureth-50/TMMG Copolymer | 0,50 | |
| Parfüm, Konservierungsmittel, | | |
| Farbstoffe usw. | q.s. | |
| Natriumhydroxid | q.s. | |
| Wasser | ad 100,00 | |
| pH-Wert eingestellt auf 6,0-7,5 | | |
| Emulsion VI | | 60 |
| Gas (Stickstoff) | | 40 |

Vordispergierung des anorganischen Gelbildners und Quellung des Hydrokolloids (quervernetztes Stärkederivat) sowie des nichtionischen Polymers (PEG-180/Laureth-50/TMMG Copolymer) unter Rühren in der Wasserphase. Vereinigung der auf 78 °C aufgeheizten Fettphase mit der auf 75 °C aufgeheizten Wasserphase. Zugabe der partikulären hydrophoben, hydrophobisierten Festkörpersubstanzen (Talkum) unter Rühren. Homogenisierung mittels einer Zahnkranzdispergiermaschine (Rotor-Stator-Prinzip) bei 65 °C. 45 min Rühren im Becomix unter Begasung mit Stickstoff bei 1 bar unter Kühlung auf 30 °C. Zugabe der Additive bei 30 °C (Parfüm, Wirkstoffe).

Homogenisierung mittels einer Zahnkranzdispergiermaschine (Rotor-Stator-Prinzip) bei 23 °C.

## Patentansprüche

1. Selbstschäumende und/oder schaumförmige kosmetische oder dermatologische Zubereitungen, welche
I. ein Emulgatorsystem, welches aus
A. mindestens einem Emulgator A, gewählt aus der Gruppe der ganz-, teil- oder nicht neutralisierten, verzweigten und/oder unverzweigten, gesättigten und/oder ungesättigten Fettsäuren mit einer Kettenlänge von 10 bis 40 Kohlenstoffatomen,
B. mindestens einem Emulgator B, gewählt aus der Gruppe der polyethoxylierten Fettsäurester mit einer Kettenlänge von 10 bis 40 Kohlenstoffatomen und mit einem Ethoxylierungsgrad von 5 bis 100 und
C. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen,
besteht,
II. bis zu 30 Gew.-% - bezogen auf das Gesamtgewicht der Zubereitung - einer Lipidphase,
III. ein oder mehrere vorgelatinisierte, quervernetzte Stärkederivate und
IV. 1 bis 90 Vol.-%, bezogen auf das Gesamtvolumen der Zubereitung, mindestens eines Gases, gewählt aus der Gruppe Luft, Sauerstoff, Stickstoff, Helium, Argon, Lachgas (N₂O) und Kohlendioxid (CO₂)
enthalten.

2. Zubereitung nach nach Anspruch 1, **dadurch gekennzeichnet, daß** die Gewichtsverhältnisse von Emulgator A zu Emulgator B zu Coemulgator C (A : B : C) wie a : b : c gewählt wird, wobei a, b und c unabhängig voneinander rationale Zahlen von 1 bis 5, bevorzugt von 1 bis 3 darstellen.

3. Zubereitung nach einem der vorhergehenden Ansprüche, **dadurch gekennzeichnet, daß** die Gewichtsverhältnisse von Emulgator A zu Emulgator B zu Coemulgator C (A : B : C) wie etwa 1 : 1 : 1gewählt wird

4. Zubereitung nach einem der vorhergehenden Ansprüche, **dadurch gekennzeichnet, daß** die Gesamtmenge der Substanzen gemäß A., B. und C. aus dem Bereich von 2 bis 20 Gew.-%, bezogen auf das Gesamtgewicht der Formulierung, gewählt werden.

5. Zubereitung nach einem der vorhergehenden Ansprüche, **dadurch gekennzeichnet, daß** sie weitere Emulgatoren, gewählt aus der Gruppe der hydrophilen Emulgatoren, insbesondere Mono-, Di-, Trifettsäureestern des Sorbitols, enthalten.

6. Zubereitung nach Anspruch 1, **dadurch gekennzeichnet, dass** als vorgelatinisierte, quervernetzte Stärkederivate hydroxypropylierte Phosphatester eingesetzt werden.

7. Zubereitung nach Anspruch 1, **dadurch gekennzeichnet, daß** als vorgelatinisierte, quervernetzte Stärkederivat Hydroxypropyldistärkephosphat (CAS Nummer 113894-92-1) eingesetzt wird

8. Zubereitung nach Anspruch 5, **dadurch gekennzeichnet, daß** die Gesamtmenge der weiteren Emulgatoren kleiner als 5 Gew.-%, bezogen auf das Gesamtgewicht der Formulierung, gewählt wird.

9. Zubereitung nach einem der vorhergehenden Ansprüche, **dadurch gekennzeichnet, daß** der Volumenanteil des oder der Gase von 10 bis 80 Vol.-%, bezogen auf das Gesamtvolumen der Zubereitung, gewählt wird.

10. Zubereitung nach einem der vorhergehenden Ansprüche, **dadurch gekennzeichnet, daß** als Gas Kohlendioxid gewählt wird.

11. Zubereitung nach einem der vorhergehenden Ansprüche, **dadurch gekennzeichnet, daß** sie eine oder mehrere Substanzen, gewählt aus der Gruppe der Moisturizer, enthält.

12. Verwendung selbstschäumender und/oder schaumförmiger kosmetischer oder dermatologischer Zubereitungen, welche
I. ein Emulgatorsystem, welches aus
A. mindestens einem Emulgator A, gewählt aus der Gruppe der ganz-, teil- oder nicht neutralisierten, verzweigten oder unverzweigten, gesättigten oder ungesättigten Fettsäuren mit einer Kettenlänge von 10 bis 40 Kohlenstoffatomen,
B. mindestens einem Emulgator B, gewählt aus der Gruppe der polyethoxylierten Fettsäurester mit einer Kettenlänge von 10 bis 40 Kohlenstoffatomen und mit einem Ethoxylierungsgrad von 5 bis 100 und
C. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen,
besteht, und
II. bis zu 30 Gew.-% - bezogen auf das Gesamtgewicht der Zubereitung - einer Lipidphase,
enthalten, als kosmetische oder dermatologische Grundlagen für gasförmige Wirkstoffe.
